# EUROPEAN PATENT SPECIFICATION

(11) **EP 1 466 987 B1**
(45) Date of publication and mention of the grant of the patent: **19.11.2008**
(21) Application number: 03700460.3
(22) Date of filing: 06.01.2003
(51) Int. Cl.: C12Q 1/32, G01N 21/78, G01N 31/00, G01N 33/50, G01N 33/66, G01N 33/92

(54) **METHOD OF COLORIMETRY**
KOLORIMETRISCHES VERFAHREN
PROCEDE DE COLORIMETRIE

(30) Priority: 28.12.2001 JP 2001400379
(43) Date of publication of application: 13.10.2004
(73) Proprietor: Arkray, Inc., Kyoto 601-8045 (JP)
(72) Inventor: NAGAKAWA, Kenji, ARKRAY, INC., Kyoto-shi, Kyoto 601-8045 (JP); TSUJIMOTO, Tomomichi, ARKRAY, INC., Kyoto-shi, Kyoto 601-8045 (JP); NISHINO, Susumu, ARKRAY, INC., Kyoto-shi, Kyoto 601-8045 (JP); TERAMOTO, Masaaki, ARKRAY, INC., Kyoto-shi, Kyoto 601-8045 (JP); KAWASE, Yoshiyuki, ARKRAY, INC., Kyoto-shi, Kyoto 601-8045 (JP)
(74) Representative: Golding, Louise Ann
(86) International application number: PCT/JP2003/000026
(87) International publication number: WO 2003/057904

(56) References cited:
- EP-A- 0 100 217
- EP-A- 1 457 572
- WO-A-94/17199
- WO-A1-92/15701
- WO-A1-94/17199
- JP-A- 1 155 244
- JP-A- 3 043 096
- US-A- 4 701 420

## Description

### Technical Field

The present invention relates to a colorimetric method and a reagent used for the same.

### Background Art

In the field of clinical or biochemical examinations, a colorimetric analysis is employed as a method for analyzing components such as glucose, cholesterol, or the like in a sample. For example, the colorimetric analysis of glucose is generally as follows: a glucose oxidase reacts with glucose (substrate) to generate gluconolactone and hydrogen peroxide; and the hydrogen peroxide is detected by a coloring reagent, such as a Trinder's reagent, in the presence of peroxidase. This method, in which the concentration of a substrate is measured indirectly via hydrogen peroxide, is not limited to glucose, but also is used to analyze other components such as cholesterol.

However, the conventional colorimetric analysis involves the following problems. First, the time required for measurement is long because it takes a long time until a color reaction ends due to the indirect measurement of an analyte via hydrogen peroxide. For example, the measurement of glucose takes 30 to 60 seconds. Second, it is difficult to set conditions because two different enzyme reaction systems should be stabilized simultaneously. Finally, the conventional colorimetric analysis requires oxygen, and inadequate oxygen supply may lead to an insufficient reaction.

### Disclosure of Invention

With the foregoing in mind, it is an object of the present invention to provide a colorimetric method that has a single reaction system and can achieve a short-time analysis and provide reliable values with the analysis.

A colorimetric method of the present invention includes allowing an oxidoreductase to act on an analyte and a transition metal complex that changes color by transferring an electron, and performing a qualitative or quantitative analysis of the analyte by measuring a color change in the transition metal complex due to electron transfer from the analyte to the transition metal complex wherein at least one ligand in the transition metal complex is selected from ammonia, a bipyridyl compound, an imidazole compound, a phenanthroline compound, an ethylenediamine compound, amino acids, a triazine compound, a biquinoline compound, a pyridylazo compound, a nitroso compound and an oxine compound.

This method has a single reaction system, so that the reaction system is simple and stable, and the time until a color reaction ends is short. Therefore, the measuring time is reduced (e.g., when glucose is used as an analyte, the measurement can be performed within about 5 seconds). Moreover, this method does not require hydrogen peroxide and in principle is not affected by the amount of oxygen, thus ensuring highly reliable values with the analysis.

A reagent for the above colorimetric method is described, that includes an oxidoreductase and a color-changeable substance that changes color by transferring an electron. A test piece may include this reagent. Compared with a conventional test piece for colorimetric analysis requiring hydrogen peroxide, said test piece can achieve a very short-time analysis and ensure highly reliable values with the analysis.

An analysis method that uses a bipyridyl metal complex as a light-emitting substance has been proposed (e.g., JP 10-253633 A). However, this method quite differs from the present invention in technical field because the present invention relates to a colorimetric method. Another technique that allows a bipyridyl metal complex to produce/erase color by direct application from an electrode has been proposed (e.g., JP 57-192483 A). However, this technique also quite differs from the present invention in technical field because the present invention utilizes electron transfer by an oxidoreductase.

EP 0100 217 discloses a process for quantitative determination of substrate treated with oxidase using an iron complex of porphyrin or an ion chelate of complexane, and a color producing reagent to be reduced.

US 4, 701, 420, WO 94/17199 and JP3043096 disclose analytic compositions comprising ferric ion chelates. Upon reduction of the ferric ion to ferrous ion, the ferrous ion complexes with a ferrous ion coordinating ligand to form a colored complex.

EP 0 602 488 and WO 96/25514 disclose electrochemical mediators comprising a transition metal and a bipyridine or phenanthroline ligand.

### Brief Description of Drawings

FIG. 1 is a graph showing the dependence of reflectance on glucose concentration in an example of the present invention.
FIG. 2 is a graph showing the dependence of reflectance on glucose concentration in another example of the present invention.
FIG. 3 is a graph showing a color change in yet another example of the present invention.
FIG. 4 is a graph showing a color change in still another example of the present invention.
FIG. 5 is a graph showing a color change in still another example of the present invention.
FIG. 6 is a graph showing a color change in still another example of the present invention.
FIG. 7 is a graph showing the dependence of reflectance on glucose concentration in still another example of the present invention.
FIGS. 8A to 8F are graphs showing a color change in still another example of the present invention.
FIGS. 9A to 9D are graphs showing a color change in still another example of the present invention.
FIGS. 10A to 10C are graphs showing a color change in still another example of the present invention.
FIG. 11 is a graph showing a color change in still another example of the present invention.
FIG. 12 is a graph showing a color change in still another example of the present invention.
FIGS. 13A to 13C are graphs showing a color change in still another example of the present invention.
FIG. 14 is a graph showing a color change in still another example of the present invention.
FIG. 15 is a graph showing a color change in still another example of the present invention.
FIGS. 16A to 16E are graphs showing a color change in still another example of the present invention.
FIGS. 17A and 17B are graphs showing a color change in still another example of the present invention.
FIGS. 18A to 18C are graphs showing a color change in still another example of the present invention.

### Best Mode for Carrying Out the Invention

In a colorimetric method of the present invention, the color-changeable substance that changes color by transferring an electron is a transition metal complex. The transition metal complex is preferably a copper complex, an iron complex, a ruthenium complex, an osmium complex, or a mixture containing at least two of these complexes. It is preferable that a coordinating atom of a ligand in the transition metal complex is at least one selected from the group consisting of nitrogen, oxygen, and sulfur. The ligand is selected from the group consisting of ammonia, a bipyridyl compound, an imidazole compound, a phenanthroline compound, an ethylenediamine compound, amino acids, a triazine compound, a biquinoline compound, a pyridylazo compound, a nitroso compound, and an oxine compound. At least one hydrogen atom that occupies a position other than the coordination position of the ligand may be replaced by a substituent. Examples of the substituent include an alkyl group, an aryl group, an allyl group, a phenyl group, a hydroxyl group, an alkoxy group, a carboxyl group, a carbonyl group, a sulfone group, a sulfonyl group, a nitro group, a nitroso group, a primary amine, a secondary amine, a tertiary amine, an amino group, an acyl group, an amido group, and a halogen group. The transition metal complex may include two or more types of ligands, i.e., it can be a mixed ligand complex.

In a colorimetric method of the present invention, the oxidoreductase is preferably a dehydrogenase or an oxidase. The reaction rate may increase with the amount of enzyme. The analyte is, e.g., glucose, cholesterol, uric acid, lactic acid, pyruvic acid, creatine, or creatinine. In this case, the oxidoreductase may be a dehydrogenase or an oxidase that corresponds to the respective analyte.

In a colorimetric method of the present invention, it is preferable that a mediator is used in addition to the color-changeable substance that changes color by transferring an electron. Although the color-changeable substance can serve as a mediator, it has the function not only of transferring an electron but also changing color. Therefore, the additional mediator differs from the color-changeable substance. The mediator increases the speed of electron transfer, which in turn increases the speed of color change of the color-changeable substance. Thus, it is possible to use less oxidoreductase and obtain a cost advantage. The mediator is, e.g., an osmium complex, a ruthenium complex, or the like. Specific examples of the mediator will be described later. When the color-changeable substance is used with the mediator, it is preferable that a copper complex is used as the color-changeable substance, and an osmium or a ruthenium complex is used as the mediator.

A test piece preferably includes an inorganic gel as well as the reagent. The color-changeable substance that changes color by transferring an electron is reduced by electron transfer and thus changes color. However, when oxygen is present in the surroundings, the color may fade due to reoxidation of the color-changeable substance. The inorganic gel can be useful to prevent the fading.

Hereinafter, the present invention will be described in more detail by way of specific examples.

As described above, the color-changeable substance that changes color by transferring an electron is a transition metal complex in the present invention. Among the transition metal complexes particularly suitable for the color-changeable substance are a copper complex, an iron complex, a ruthenium complex, and an osmium complex.

### Copper complex

The color of the copper complex is changed, e.g., from blue (Cu²⁺) to reddish brown (Cu⁺) by electron transfer that is caused by an enzyme. Examples of a ligand in the copper complex include ammonia, a bipyridyl compound, an imidazole compound, a phenanthroline compound, an ethylenediamine compound, amino acids, a triazine compound, a biquinoline compound, a pyridylazo compound, a nitroso compound, an oxine compound, a benzothiazole compound, an acetylacetone compound, an anthraquinone compound, a xanthene compound, oxalic acid, and a derivative of each of the compounds. A mixed ligand with two or more types of these ligands may be used.

For the bipyridyl compound, the coordination number is 4 or 6. In view of stability, two bipyridyls should be coordinated at two coordination positions in the complex, respectively. Hydrogen atoms binding to the pyridine rings may be unsubstituted or substituted. The introduction of a substituent makes it possible to control, e.g., the solubility and oxidation-reduction potential of the complex. Examples of the position of the substituent include the 4,4'-position and 5,5'-position. Examples of the substituent include an alkyl group (such as a methyl group, an ethyl group, or a propyl group), an aryl group, an allyl group, a phenyl group, a hydroxyl group, an alkoxy group (such as a methoxy group or an ethoxy group), a carboxyl group, a carbonyl group, a sulfone group, a sulfonyl group, a nitro group, a nitroso group, a primary amine, a secondary amine, a tertiary amine, an amino group, an acyl group, an amido group, and a halogen group (such as bromine, chlorine, or iodine).

Examples of a bipyridyl copper complex include [Cu(bipyridyl)₂], [Cu(4,4'-dimethyl-2,2'-bipyridyl₂], [Cu(4,4'-diphenyl-2,2'-bipyridyl)₂], [Cu(4,4'-diamino-2,2'-bipyridyl)₂], [Cu(4,4'-dihydroxy-2,2'-bipyridyl)₂], [Cu(4,4'-dicarboxy-2,2'-bipyridyl)₂], [Cu(4,4'-dibromo-2,2'-bipyridyl)₂], [Cu(5,5'-dimethyl-2,2'-bipyridyl)₂], [Cu(5,5'-diphenyl-2,2'-bipyridy)₂], [Cu(5,5'-diamino-2,2'-bipyridyl)₂], [Cu(5,5'-dihydroxy-2,2'-bipyridyl)₂], [Cu(5,5'-dicarboxy-2,2'-bipyridyl)₂], [Cu(5,5'-dibromo-2,2'-bipyridyl)₂], [Cu(bipyridyl)₃], [Cu(4,4'-dimethyl-2,2'-bipyridyl)₃], [Cu(4,4'-diphenyl-2,2'-bipyridyl)₃], [Cu(4,4'-diamino-2,2'-bipyridyl)₃], [Cu(4,4'-dihydroxy-2,2'-bipyridyl)₃], [Cu(4,4'-dicarboxy-2,2'-bipyridyl)₃], [Cu(4,4'-dibromo-2,2'-bipyridyl)₃], [Cu(5,5'-dimethyl-2,2'-bipyridyl)₃], [Cu(5,5'-diphenyl-2,2'-bipyridyl)₃], [Cu(5,5'-diamino-2,2'-bipyridyl)₃], [Cu(5,5'-dihydroxy-2,2'-bipyridyl)₃], [Cu(5,5'-dicarboxy-2,2'-bipyridyl)₃], and [Cu(5,5'-dibromo-2,2'-bipyridyl)₃].

For the imidazole compound, the coordination number is 4. Hydrogen atoms binding to the imidazole rings may be unsubstituted or substituted. The introduction of a substituent makes it possible to control, e.g., the solubility and oxidation-reduction potential of the complex. Examples of the position of the substituent include the 2-position, 4-position and 5-position. Examples of the substituent include an alkyl group (such as a methyl group, an ethyl group, or a propyl group), an aryl group, an allyl group, a phenyl group, a hydroxyl group, an alkoxy group (such as a methoxy group or an ethoxy group), a carboxyl group, a carbonyl group, a sulfone group, a sulfonyl group, a nitro group, a nitroso group, a primary amine, a secondary amine, a tertiary amine, an amino group, an acyl group, an amido group, and a halogen group (such as bromine, chlorine, or iodine).

Examples of an imidazole copper complex include [Cu(imidazole)₄], [Cu(4-methyl-imidazole)₄], [Cu(4-phenyl-imidazole)₄], [Cu(4-amino-imidazole)₄], [Cu(4-hydroxy-imidazole)₄], [Cu(4-carboxy-imidazole)₄], and [Cu(4-bromo-imidazole)₄].

The amino acids include, e.g., arginine (L-Arg). A copper complex containing arginine generally has the advantage of high solubility. The mixed ligand may be a combination of the bipyridyl compounds and the imidazole compounds or a combination of the bipyridyl compounds and the amino acids, such as [Cu(imidazole)₂(bipyridyl)] or [Cu(L-Arg)₂(bipyridyl)]. The mixed ligand can be used to impart various properties to the complex, e.g., arginine improves the solubility of the complex.

### Iron complex

The color of the iron complex is changed, e.g., from yellow (Fe³⁺) to red (Fe²⁺) by electron transfer that is caused by an enzyme. Examples of a ligand in the iron complex include ammonia, a bipyridyl compound, an imidazole compound, a phenanthroline compound, an ethylenediamine compound, amino acids, a triazine compound, a biquinoline compound, a pyridylazo compound, a nitroso compound, an oxine compound, a benzothiazole compound, an acetylacetone compound, an anthraquinone compound, a xanthene compound, oxalic acid, and a derivative of each of the compounds. A mixed ligand with two or more types of these ligands may be used.

For the bipyridyl compound, the coordination number is 6. Hydrogen atoms binding to the pyridine rings may be unsubstituted or substituted. The introduction of a substituent makes it possible to control, e.g., the solubility and oxidation-reduction potential of the complex. Examples of the position of the substituent include the 4,4'-position and 5,5'-position. Examples of the substituent include an alkyl group (such as a methyl group, an ethyl group, or a propyl group), an aryl group, an allyl group, a phenyl group, a hydroxyl group, an alkoxy group (such as a methoxy group or an ethoxy group), a carboxyl group, a carbonyl group, a sulfone group, a sulfonyl group, a nitro group, a nitroso group, a primary amine, a secondary amine, a tertiary amine, an amino group, an acyl group, an amido group, and a halogen group (such as bromine, chlorine, or iodine).

Examples of a bipyridyl iron complex include [Fe(bipyridyl)₃], [Fe(4,4'-dimethyl-2,2'-bipyridyl)₃], [Fe(4,4'-diphenyl-2,2'-bipyridyl)₃], [Fe(4,4'-diamino-2,2'-bipyridyl)₃], [Fe(4,4'-dihydroxy-2,2'-bipyridyl)₃], [Fe(4,4'-dicarboxy-2,2'-bipyridyl)₃], [Fe(4,4'-dibromo-2,2'-bipyridyl)₃], [Fe(5,5'-dimethyl-2,2'-bipyridyl)₃], [Fe(5,5'-diphenyl-2,2'-bipyridyl)₃], [Fe(5,5'-diamino-2,2'-bipyridyl)₃], [Fe(5,5'-dihydroxy-2,2'-bipyridy)₃], [Fe(5,5'-dicarboxy-2,2'-bipyridyl)₃], and [Fe(5,5'-dibromo-2,2'-bipyridyl)₃].

For the imidazole compound, the coordination number is 6. Hydrogen atoms binding to the imidazole rings may be unsubstituted or substituted. The introduction of a substituent makes it possible to control, e.g., the solubility and oxidation-reduction potential of the complex. Examples of the position of the substituent include the 2-position, 4-position and 5-position. Examples of the substituent include an alkyl group (such as a methyl group, an ethyl group, or a propyl group), an aryl group, an allyl group, a phenyl group, a hydroxyl group, an alkoxy group (such as a methoxy group or an ethoxy group), a carboxyl group, a carbonyl group, a sulfone group, a sulfonyl group, a nitro group, a nitroso group, a primary amine, a secondary amine, a tertiary amine, an amino group, an acyl group, an amido group, and a halogen group (such as bromine, chlorine, or iodine).

Examples of an imidazole iron complex include [Fe(imidazole)₆], [Fe(4-methyl-imidazole)₆], [Fe(4-phenyl-imidazole)₆], [Fe(4-amino-imidazole)₆], [Fe(4-hydroxy-imidazole)₆], [Fe(4-carboxy-imidazole)₆], and [Fe(4-bromo-imidazole)₆].

The amino acids include, e.g., arginine (L-Arg). An iron complex containing arginine generally has the advantage of high solubility. The mixed ligand may be a combination of the bipyridyl compounds and the imidazole compounds or a combination of the bipyridyl compounds and the amino acids, such as [Fe(imidazole)₂(bipyridyl)₂] or [Fe(L-Arg)₂(bipyridyl)₂]. The mixed ligand can be used to impart various properties to the complex, e.g., arginine improves the solubility of the complex.

### Ruthenium complex

Examples of a ligand in the ruthenium complex include ammonia, a bipyridyl compound, an imidazole compound, a phenanthroline compound, an ethylenediamine compound, amino acids, a triazine compound, a biquinoline compound, a pyridylazo compound, a nitroso compound, an oxine compound, a benzothiazole compound, an acetylacetone compound, an anthraquinone compound, a xanthene compound, oxalic acid, and a derivative of each of the compounds. A mixed ligand with two or more types of these ligands may be used.

For the bipyridyl compound, the coordination number is 6. Hydrogen atoms binding to the pyridine rings may be unsubstituted or substituted. The introduction of a substituent makes it possible to control, e.g., the solubility and oxidation-reduction potential of the complex. Examples of the position of the substituent include the 4,4'-position and 5,5'-position. Examples of the substituent include an alkyl group (such as a methyl group, an ethyl group, or a propyl group), an aryl group, an allyl group, a phenyl group, a hydroxyl group, an alkoxy group (such as a methoxy group or an ethoxy group), a carboxyl group, a carbonyl group, a sulfone group, a sulfonyl group, a nitro group, a nitroso group, a primary amine, a secondary amine, a tertiary amine, an amino group, an acyl group, an amido group, and a halogen group (such as bromine, chlorine, or iodine).

Examples of a bipyridyl ruthenium complex include [Ru(bipyridyl)₃], [Ru(4,4'-dimethyl-2,2'-bipyridyl)₃], [Ru(4,4'-diphenyl-2,2'-bipyridyl)₃], [Ru(4,4'-diamino-2,2'-bipyridyl)₃], [Ru(4,4'-dihydroxy-2,2'-bipyridyl)₃], [Ru(4,4'-dicarboxy-2,2'-bipyridyl)₃], [Ru(4,4'-dibromo-2,2'-bipyridyl)₃], [Ru(5,5'-dimethyl-2,2'-bipyridyl)₃], [Ru(5,5'-diphenyl-2,2'-bipyridyl)₃], [Ru(5,5'-diamino-2,2'-bipyridyl)₃], [Ru(5,5'-dihydroxy-2,2'-bipyridyl)₃], [Ru(5,5'-dicarboxy-2,2'-bipyridyl)₃], and [Ru(5,5'-dibromo-2,2'-bipyridyl)₃].

For the imidazole compound, the coordination number is 6. Hydrogen atoms binding to the imidazole rings may be unsubstituted or substituted. The introduction of a substituent makes it possible to control, e.g., the solubility and oxidation-reduction potential of the complex. Examples of the position of the substituent include the 2-position, 4-position and 5-position. Examples of the substituent include an alkyl group (such as a methyl group, an ethyl group, or a propyl group), an aryl group, an allyl group, a phenyl group, a hydroxyl group, an alkoxy group (such as a methoxy group or an ethoxy group), a carboxyl group, a carbonyl group, a sulfone group, a sulfonyl group, a nitro group, a nitroso group, a primary amine, a secondary amine, a tertiary amine, an amino group, an acyl group, an amido group, and a halogen group (such as bromine, chlorine, or iodine).

Examples of an imidazole ruthenium complex include [Ru(imidazole)₆], [Ru(4-methyl-imidazole)₆], [Ru(4-phenyl-imidazole)₆] [Ru(4-amino-imidazole)₆], [Ru(4-hydroxy-imidazole)₆], [Ru(4-carboxy-imidazole)₆], and [Ru(4-bromo-imidazole)₆].

The amino acids include, e.g., arginine (L-Arg). A ruthenium complex containing arginine generally has the advantage of high solubility. The mixed ligand may be a combination of the bipyridyl compounds and the imidazole compounds or a combination of the bipyridyl compounds and the amino acids, such as [Ru(imidazole)₂(bipyridy)₂] or [Ru(L-Arg)₂(bipyridyl)₂]. The mixed ligand can be used to impart various properties to the complex, e.g., arginine improves the solubility of the complex.

### Osmium complex

The color of the osmium complex is changed, e.g., from orange (Os³⁺) to dark brown (Os²⁺) by electron transfer that is caused by an enzyme. Examples of a ligand in the osmium complex include ammonia, a bipyridyl compound, an imidazole compound, a phenanthroline compound, an ethylenediamine compound, amino acids, a triazine compound, a biquinoline compound, a pyridylazo compound, a nitroso compound, an oxine compound, a benzothiazole compound, an acetylacetone compound, an anthraquinone compound, a xanthene compound, oxalic acid, and a derivative of each of the compounds. A mixed ligand with two or more types of these ligands may be used.

For the bipyridyl compound, the coordination number is 6. Hydrogen atoms binding to the pyridine rings may be unsubstituted or substituted. The introduction of a substituent makes it possible to control, e.g., the solubility and oxidation-reduction potential of the complex. Examples of the position of the substituent include the 4,4'-position and 5,5'-position. Examples of the substituent include an alkyl group (such as a methyl group, an ethyl group, or a propyl group), an aryl group, an allyl group, a phenyl group, a hydroxyl group, an alkoxy group (such as a methoxy group or an ethoxy group), a carboxyl group, a carbonyl group, a sulfone group, a sulfonyl group, a nitro group, a nitroso group, a primary amine, a secondary amine, a tertiary amine, an amino group, an acyl group, an amido group, and a halogen group (such as bromine, chlorine, or iodine).

Examples of a bipyridyl osmium complex include [Os(bipyridyl)₃], [Os(4,4'-dimethyl-2,2'-bipyridyl)₃], [Os(4,4'-diphenyl-2,2'-bipyridyl)₃], [Os(4,4'-diamino-2,2'-bipyridyl)₃], [Os(4,4'-dihydroxy-2,2'-bipyridyl)₃], [Os(4,4'-dicarboxy-2,2'-bipyridyl)₃], [Os(4,4'-dibromo-2,2'-bipyridyl)₃], [Os(5,5'-dimethyl-2,2'-bipyridyl)₃], [Os(5,5'-diphenyl-2,2'-bipyridyl)₃], [Os(5,5'-diamino-2,2'-bipyridyl)₃], [Os(5,5'-dihydroxy-2,2'-bipyridyl)₃], [Os(5,5'-dicarboxy-2,2'-bipyridyl)₃], and [Os(5,5'-dibromo-2,2'-bipyridyl)₃].

For the imidazole compound, the coordination number is 6. Hydrogen atoms binding to the imidazole rings may be unsubstituted or substituted. The introduction of a substituent makes it possible to control, e.g., the solubility and oxidation-reduction potential of the complex. Examples of the position of the substituent include the 2-position, 4-position and 5-position. Examples of the substituent include an alkyl group (such as a methyl group, an ethyl group, or a propyl group), an aryl group, an allyl group, a phenyl group, a hydroxyl group, an alkoxy group (such as a methoxy group or an ethoxy group), a carboxyl group, a carbonyl group, a sulfone group, a sulfonyl group, a nitro group, a nitroso group, a primary amine, a secondary amine, a tertiary amine, an amino group, an acyl group, an amido group, and a halogen group (such as bromine, chlorine, or iodine).

Examples of an imidazole osmium complex include [Os(imidazole)₆], [Os(4-methyl-imidazole)₆], [Os(4-phenyl-imidazole)₆], [Os(4-amino-imidazole)₆], [Os(4-hydroxy-imidazole)₆], [Os(4-carboxy-imidazole)₆], and [Os(4-bromo-imidazole)₆].

The amino acids include, e.g., arginine (L-Arg). An osmium complex containing arginine generally has the advantage of high solubility. The mixed ligand may be a combination of the bipyridyl compounds and the imidazole compounds or a combination of the bipyridyl compounds and the amino acids, such as [Os(imidazole)₂(bipyridyl)₂] or [Os(L-Arg)₂(bipyridyl)₂]. The mixed ligand can be used to impart various properties to the complex, e.g., arginine improves the solubility of the complex.

The above explanation of the transition metal complexes is based on the type of transition metal, and the present invention is not limited thereto. Hereinafter, the transition metal complexes will be described based on their ligands.

A ligand that contains coordinating atoms N, O, and S has groups such as =N-OH, -COOH, -OH, -SH, and >C=O in the molecule. Examples of metal complexes including this type of ligand are NN chelate, NO chelate, NS chelate, OO chelate, OS chelate, SS chelate (bidentate), N chelate (unidentate), and NNN chelate (tridentate). The combination is diverse. When a ligand has a double bond, Cu, Fe, Ru, and Os of the complex tend to have the function of transferring electrons. The ligand preferably has an aromatic ring. The ligand may be any of the above substituents. For example, the introduction of a sulfone group can improve the solubility of the metal complex. The metal complex may be formed by mixing two or more types of ligands and used as a mixed ligand complex. For example, when one of the ligands is amino acids, the metal complex may have a good affinity with an enzyme. Moreover, various halogen atoms (such as Cl, F, Br, and I) can be attached to part of the site of the central metal. The following is an example of the transfer metal complexes that are classified by the type of coordination.
*NN coordination form*
   Phenanthroline derivative
   Cu + 1,10-phenanthroline
   Fe + 1,10-phenanthroline
   Cu + bathophenanthroline
   Fe + bathophenanthroline
   Cu + bathophenanthroline sulfonic acid
   Fe + bathophenanthroline sulfonic acid
   Bipyridyl derivative
   Cu + 2,2'-bipyridyl
   Fe + 2,2'-bipyridyl
   Fe + 4,4'-diamino-2,2'-bipyridyl
   Ru + 4,4'-diamino-2,2'-bipyridyl
   Triazine derivative
   Cu + TPTZ (2,4,6-tripyridyl-S-triazine)
   Fe + TPTZ (2,4,6-tripyridyl-S-triazine)
   Fe+PDTS (3-(2-pyridyl)-5,6-bis(4-sulfophenyl)-1,2,4-triazine)
   Biquinoline derivative
   Cu + cuproin (2,2'-biquinoline)
   Pyridylazo derivative
   Fe + nitro-PAPS (2-(5-nitro-2-pyridylazo)-5-[N-n-propyl-N-(3-sulfopropyl) amino] phenol)
*NO coordination form*
   Fe + nitroso-PSAP (2-nitroso-5-[N-n-propyl-N-(3-sulfopropyl) amino] phenol)
   Fe + nitroso-ESAP (2-nitroso-5-[N-ethyl-N-(3-sulfopropyl) amino] phenol) Fe + 1-nitroso-2-naphthol
*NS coordination form*
   Fe + 2-amino-4-thiazole acetic acid
*OO coordination form*
   Fe + 1,2-naphthoquinone-4-sulfonic acid
*Mixed ligand form*
   Os + Cl, imidazole, 4,4'-dimethyl-2,2'-bipyridyl
   Os + imidazole, 4,4'-dimethyl-2,2'-bipyridyl
   Cu + L-arginine, 2,2'-bipyridyl
   Cu + ethylenediamine, 2,2'-bipyridyl
   Cu + imidazole, 2,2'-bipyridyl

Next, the colorimetric method of the present invention is applied to a test piece. In this case, a copper complex is used as the color-changeable substance that changes color by transferring an electron, and glucose is used as the analyte. Other analytes such as cholesterol are analyzed basically in the same manner except that the oxidoreductase is changed in accordance with the analytes.

First, a bipyridyl copper complex is prepared either by using a commercially available product or by employing the following manner. For example, CuCl₂ and 2,2'-bipyridyl (bpy) are mixed in a water bath at about 60°C to 90°C and synthesized into [Cu(bpy)₂]Cl₂. The molar ratio of CuCl₂ to 2,2'-bipyridyl (bpy) is, e.g., 1 : 2. The concentration of the aqueous solution of bipyridyl copper complex ranges, e.g., from 1 to 10 wt%. A binder is dissolved in the aqueous solution of bipyridyl copper complex, and then glucose dehydrogenase (GDH) is dissolved in the binder solution, thus producing a reagent solution. Examples of the binder include hydroxypropylcellulose (HPC), polyvinyl alcohol (PVA), polyvinyl pyrrolidone (PVP), polyacrylamide, and bovine serum albumin (BSA), and HPC is preferred. The concentration of the binder ranges, e.g., from 0.5 to 5 wt%. The concentration of GDH ranges, e.g., from 1000 to 50000 U/ml. A porous sheet (e.g., a filter paper) is impregnated with the reagent solution and dried, so that a test piece for glucose analysis can be produced. Before impregnation of the reagent solution, it is preferable that the porous sheet is impregnated with an inorganic gel solution and dried. The inorganic gel can be smectite or the like. The concentration of the inorganic gel in the solution ranges, e.g., from 1 to 5 wt%, preferably 1 to 3 wt%, and more preferably 1.5 to 2 wt%. The inorganic gel solution also may include an amphoteric surfactant such as CHAPS. The concentration of the amphoteric surfactant with respect to the total inorganic gel solution ranges, e.g., from 0.1 to 2 wt%, preferably 0.1 to 1 wt%, and more preferably 0.2 to 0.4 wt%. The amount of inorganic gel impregnated into the porous sheet ranges, e.g., from 1 to 50 mg/cm³, preferably 10 to 30 mg/cm³, and more preferably 15 to 20 mg/cm³, when measured on the basis of the volume of voids in the porous sheet. The porous sheet can be an asymmetrical porous film in which pore sizes vary in the thickness direction or in the sheet surface direction. The original color of this test piece is blue. When a sample containing glucose (e.g., blood) is applied to the test piece, the color changes to reddish brown in accordance with the glucose concentration. Therefore, the qualitative or quantitative analysis of the glucose can be performed using the color change. The time required for the analysis is about 2 to 3 seconds after the application of the sample. If the test piece is impregnated with an inorganic gel, it is possible to prevent fading caused by reoxidation after the color change and, e.g., to increase the time from the end of a color reaction to the start of measuring the color thus produced.

The inorganic gel is preferably selected from swelling clay minerals. Among the swelling clay minerals, bentonite, smectite, vermiculite, or synthetic fluorine mica is more preferred. In particular, synthetic smectite such as synthetic hectorite or synthetic saponite, or synthetic mica (the natural mica generally is a non-swelling clay mineral) such as swelling synthetic mica (or Na mica) typified by synthetic fluorine mica is preferred.

Next, the colorimetric method of the present invention is applied to liquid system analysis. In this case, a copper complex is used as the color-changeable substance that changes color by transferring an electron, and glucose is used as the analyte. Other analytes such as cholesterol are analyzed basically in the same manner except that the oxidoreductase is changed in accordance with the analytes.

First, a copper complex [Cu(bpy)₂]Cl₂ is synthesized in such a manner as described above. Then, a reagent solution is prepared by dissolving the copper complex and GDH in a buffer solution. Although they may be dissolved in water, the buffer solution is preferred. The pH of the buffer solution ranges, e.g., from 6 to 8, and preferably 6.5 to 7. The concentration of the copper complex ranges, e.g., from 0.1 to 60 mM, preferably 0.2 to 10 mM, and more preferably 0.3 to 0.6 mM. The concentration of GDH ranges, e.g., from 10 to 1000 U/ml, preferably 50 to 500 U/ml, and more preferably 100 to 200 U/ml. When a sample containing glucose (e.g., blood) is added to the reagent solution, the color of the reagent solution changes from blue to reddish brown in accordance with the glucose concentration in a short time, e.g., 5 seconds or less. This change may be observed visually or measured with an optical measuring device such as a spectrophotometer. The amount of the added sample ranges, e.g., from 1 to 100 µl, preferably 3 to 10 µl, and more preferably 5 to 10 µl with respect to 1 ml of the reagent solution.

As described above, it is preferable that a mediator such as an osmium complex or a ruthenium complex is used in addition to the color-changeable substance that changes color by transferring an electron in the colorimetric method of the present invention. For a test piece, the amount of mediator with respect to the total reagent solution ranges, e.g., from 0.1 to 50 mM, preferably 0.5 to 10 mM, and more preferably 1 to 3 mM. For liquid system analysis, the amount of mediator with respect to the total reagent solution ranges, e.g., from 0.1 to 10 mM, preferably 0.1 to 1 mM, and more preferably 0.1 to 0.3 mM. These ranges of optimum concentration depend on the type of mediator to be used.

### Examples

Hereinafter, examples of the present invention will be described. In each of the examples, PQQ represents pyrroloquinoline quinone, and other reagents are explained in detail in the following table.

| Reagent | Manufacturer | Note (name, etc.) |
|---|---|---|
| PQQGDH | TOYOBO Co., Ltd | PQQ-glucose dehyrogenase |
| GOD | Sigma | Glucose oxidase type X-S |
| Pyruvate oxidase | BoehringerMannheim | |
| Glucose | Wako Pure Chemical Industries, Ltd. | D(+)-glucose |
| Pyruvic acid | Wako Pure Chemical Industries, Ltd. | Lithium pyruvate monohydrate |

### Example 1

An aqueous solution of [Cu(bpy)₂]Cl₂-6H₂O (80 mM) was prepared by mixing CuCl₂ and 2,2'-bipyridyl at a molar ratio of 1 : 2 in a water bath at about 80°C. HPC-M was dissolved in the aqueous solution of bipyridyl copper complex at 2 wt%, and then was heated to 50°C and cooled to 25°C. Further, GDH was dissolved in this aqueous solution at 50000 U/ml, thus producing a reagent solution. Next, an asymmetrical porous film ("BTS-25", manufactured by US Filter) in which pore sizes vary in the thickness direction was impregnated from the surface that includes smaller pores with 2 wt% of an aqueous solution of inorganic gel ("Laponite XLG", manufactured by Rockwood Additives Limited), and then was dried. Moreover, 2 µl of the reagent solution was applied to the surface of the porous film that includes larger pores, and then was air-dried to form a circular spot (light blue). This spot portion was cut and sandwiched between PET films with holes, so that an intended test piece for glucose analysis was obtained.

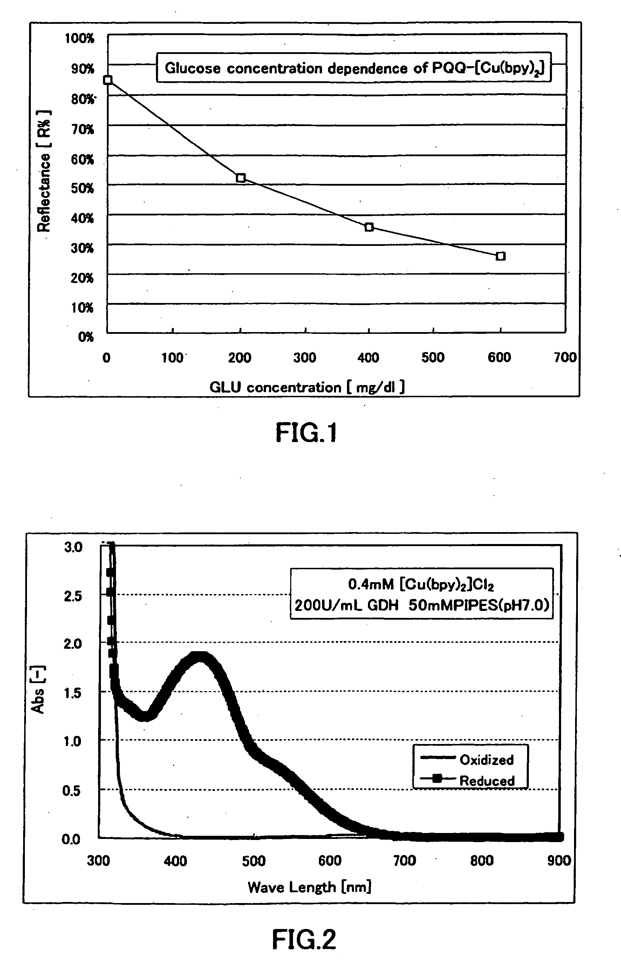

Serum having four different glucose concentrations (0 mg/ml, 2 mg/ml, 4 mg/ml, and 6 mg/ml) was applied to the test piece, and color produced in the test piece was measured after 5 seconds from the application by using a reflectance measuring device (wavelength: 470 nm). The graph in FIG. 1 shows the results. As shown in FIG. 1, the test piece produced color in accordance with the glucose concentration within 5 seconds from the application. This color (reddish brown) corresponding to the glucose concentration also was observed visually. The time required for producing the color was 2 to 3 seconds. The glucose-containing serum was prepared in the following manner. Human blood plasma was glycolyzed completely, frozen, and melted to produce serum. Then, glucose was added to the serum in different concentrations as described above.

### Example 2

Copper (II) chloride (0.01 mol) was dissolved in hot water (30 ml), to which 2,2'-bipyridyl (0.02 mol) was added and stirred. Then, the solution was cooled to precipitate hexahydrate as crystals, thus producing [Cu(bpy)₂]Cl₂·6H₂O. The following reaction reagent (1 ml) including this copper complex was placed in a microcell having an optical path length of 10 mm, and the absorption spectrum at a wavelength of 300 nm to 900 nm was measured with a spectrophotometer ("V-550", manufactured by JASCO Corporation) and identified as a blank (oxidized). A 500 mM glucose aqueous solution (10 µl) was added to the microcell while stirring, and the spectrum was measured immediately. The graph in FIG. 2 shows the results. As shown in FIG. 2, the copper complex was reduced by the enzyme reaction, and the color production was observed in a short wavelength region.

| Reaction reagent composition | |
|---|---|
| [Cu(bpy)₂]Cl₂ | 0.4 mM |
| PIPES (pH 7.0) | 50 mM |
| PQQGDH | 200 U/ml |

### Example 3

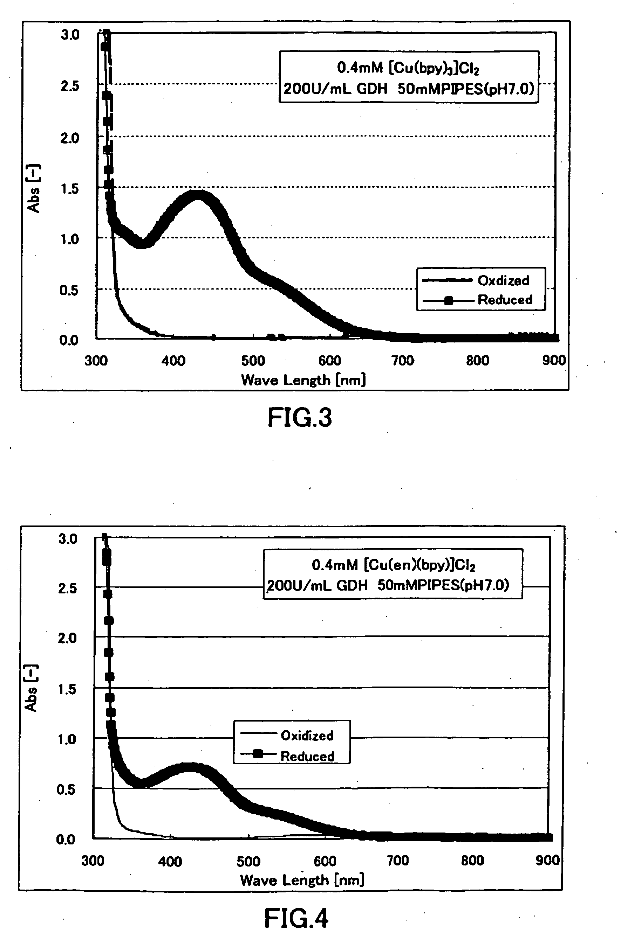

Copper (II) chloride (0.01 mol) and 2,2'-bipyridyl (0.033 mol) were added to a small amount of water and heated until they were dissolved completely. Then, the solution was cooled to precipitate [Cu(bpy)₃]Cl₂·6H₂O as crystals. The following reaction reagent (1 ml) including this copper complex was placed in a microcell having an optical path length of 10 mm, and the absorption spectrum at a wavelength of 300 nm to 900 nm was measured with a spectrophotometer ("V-550", manufactured by JASCO Corporation) and identified as a blank (oxidized). A 500 mM glucose aqueous solution (10 µl) was added to the microcell while stirring, and the spectrum was measured immediately. The graph in FIG. 3 shows the results. As shown in FIG. 3, the copper complex was reduced by the enzyme reaction, and the color production was observed in a short wavelength region.

| Reaction reagent composition | |
|---|---|
| [Cu(bpy)₃]Cl₂ | 0.4 mM |
| PIPES (pH 7.0) | 50 mM |
| PQQGDH | 200 U/ml |

### Example 4

Copper (II) chloride (0.01 mol) was dissolved in hot water (10 ml), ethylenediamine (en, 0.01 mol) was dissolved in hot water (10 ml), and 2,2'-bipyridyl (0.01 mol) was dissolved in hot ethanol (10 ml). The three solutions were mixed together to make a blue solution. This solution was cooled and concentrated, thus producing needle crystals of [Cu(en)(bpy)]Cl₂. The following reaction reagent (1 ml) including this copper complex was placed in a microcell having an optical path length of 10 mm, and the absorption spectrum at a wavelength of 300 nm to 900 nm was measured with a spectrophotometer ("V-550", manufactured by JASCO Corporation) and identified as a blank (oxidized). A 500 mM glucose aqueous solution (10 µl) was added to the microcell while stirring, and the spectrum was measured immediately. The graph in FIG. 4 shows the results. As shown in FIG. 4, the copper complex was reduced by the enzyme reaction, and the color production was observed in a short wavelength region.

| Reaction reagent | |
|---|---|
| [Cu(en)(bpy)]Cl₂ | 0.4 mM |
| PIPES (pH 7.0) | 50 mM |
| PQQGDH | 200 U/ml |

### Example 5

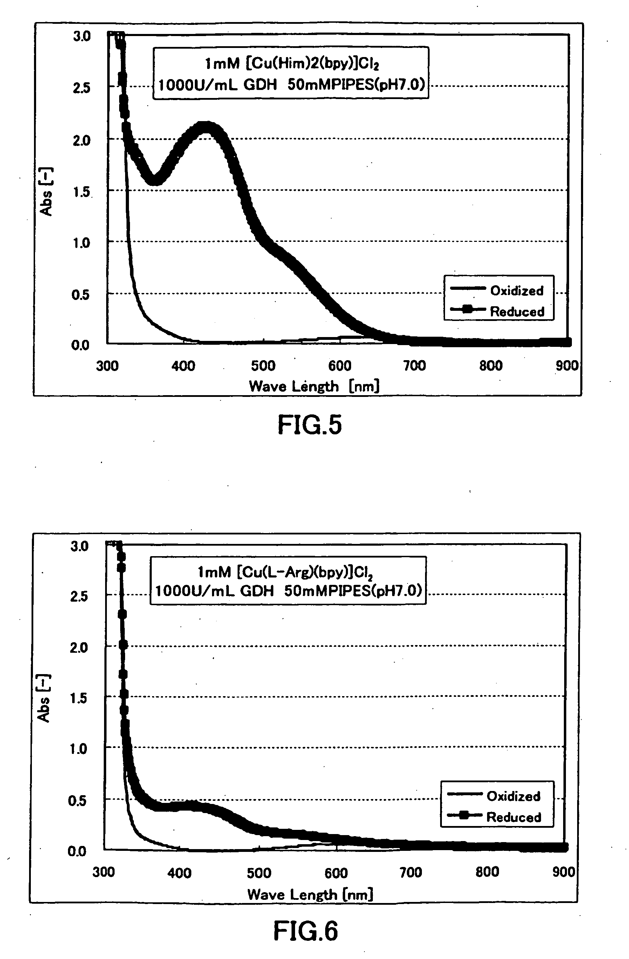

An aqueous solution of copper chloride was prepared by dissolving 511 mg (3.0 mmol, 1.0 eq.) of copper (II) chloride dihydrate in hot water (10 mL). Further, 408 mg (6.0 mmol, 2.0 eq.) of imidazole was dissolved in water (10 mL) and 69 mg (3.0 mmol, 1.0 eq.) of 2,2'-bipyridyl was dissolved in ethanol (10 mL), and then the two solutions were mixed together. This mixed solution was added to the aqueous solution of copper chloride, thus producing a deep blue solution of [Cu(Him)₂(bpy)]Cl₂. The following reaction regent (1 ml) including this copper complex was placed in a microcell having an optical path length of 10 mm, and the absorption spectrum at a wavelength of 300 nm to 900 nm was measured with a spectrophotometer ("V-550", manufactured by JASCO Corporation) and identified as a blank (oxidized). A 500 mM glucose aqueous solution (10 µl) was added to the microcell while stirring, and the spectrum was measured immediately. The graph in FIG. 5 shows the results. As shown in FIG. 5, the copper complex was reduced by the enzyme reaction, and the color production was observed in a short wavelength region.

| Reaction reagent | |
|---|---|
| [Cu(Him)₂(bpy)]Cl₂ | 1 mM |
| PIPES (pH 7.0) | 50 mM |
| PQQGDH | 1000 U/ml |

### Example 6

Copper (II) chloride (0.01 mol) was dissolved in hot water (10 ml), L-arginine (0.01 mol) was dissolved in hot water (10 ml), and 2,2'-bipyridyl (0.01 mol) was dissolved in hot ethanol (10 ml). The three solutions were mixed together to make a deep blue solution. This solution was cooled and concentrated, thus producing needle crystals of [Cu(L-Arg)(bpy)]Cl₂. The following reaction reagent (1 ml) including this copper complex was placed in a microcell having an optical path length of 10 mm, and the absorption spectrum at a wavelength of 300 nm to 900 nm was measured with a spectrophotometer ("V-550", manufactured by JASCO Corporation) and identified as a blank (oxidized). A 500 mM glucose aqueous solution (10 µl) was added to the microcell while stirring, and the spectrum was measured immediately. The graph in FIG. 6 shows the results. As shown in FIG. 6, the copper complex was reduced by the enzyme reaction, and the color production was observed in a short wavelength region.

| Reaction reagent | |
|---|---|
| [Cu(L-Arg)(bpy)]Cl₂ | 1 mM |
| PIPES (pH 7.0) | 50 mM |
| PQQGDH | 1000 U/ml |

### Example 7

An asymmetrical porous film ("BTS-25", manufactured by US Filter) in which pore sizes vary in the thickness direction was impregnated from the surface that includes smaller pores with 2 wt% of an aqueous solution of inorganic gel having the following composition, and then was dried. Moreover, 2 µl of a reagent solution having the following composition was applied to the surface of the porous film that includes larger pores, and then was air-dried to form a circular spot (light blue). This spot portion was cut and sandwiched between PET films with holes, so that an intended test piece for glucose analysis was obtained.

| Inorganic gel aqueous solution composition | |
|---|---|
| Smectite (which is the same as that used in Example 1) | |
| | 1.8 wt% |
| CHAPS (surface-active agent) | 0.4 wt% |
| Reagent solution composition | |
| [Cu(bpy)₂]Cl₂ | 80 mM |
| [OsCl(Him)(dmbpy)₂]Cl₃ | 3 mM |
| PQQ-GDH | 1000 U/ml |
| BSA | 5% |
| CHAPS | 0.4% |

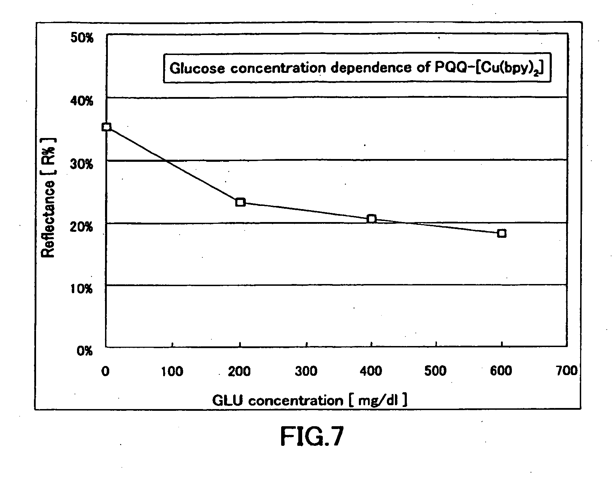

Serum having four different glucose concentrations (0 mg/ml, 2 mg/ml, 4 mg/ml, and 6 mg/ml) was applied to the test piece, and color produced in the test piece was measured after 5 seconds from the application by using a reflectance measuring device (wavelength: 470 nm). The graph in FIG. 7 shows the results. As shown in FIG. 7, the test piece produced color in accordance with the glucose concentration immediately after the application. This color (reddish brown) corresponding to the glucose concentration also was observed visually. Moreover, the color production occurred in an instant. The glucose-containing serum was prepared in the following manner. Human blood plasma was glycolyzed completely, frozen, and melted to produce serum. Then, glucose with different concentrations as described above was added to the serum.

### Example 8

A 500 mM glucose aqueous solution (10 µl) was added to a reagent solution (100 µl) having the following composition. Then, a change in color of the reagent solution from green (the original color) to reddish brown was observed visually within one minute. The original color of the reagent solution was made by mixing the yellow of a coenzyme (FAD) included in GOD and the blue of copper.

| Reagent solution composition | |
|---|---|
| GOD (manufactured by Sigma, specific activity 209 U/mg) | |
| | 50 mg/ml |
| [Cu(bpy)₂]Cl₂ | 40 mM |
| PIPES (pH 7.0) | 50 mM |
| CHAPS | 0.1 wt% |

### Example 9

A 500 mM glucose aqueous solution (10 µl) was added to a reagent solution (100 µl) having the following composition. Then, a change in color of the reagent solution from green (the original color) to reddish brown was observed visually within 5 seconds. The original color of the reagent solution was made by mixing the yellow of a coenzyme (FAD) included in GOD and the blue of copper.

| Reagent solution composition | |
|---|---|
| GOD (manufactured by Sigma, specific activity 209 U/mg) | |
| | 50 mg/ml |
| [Cu(bpy)₂]Cl₂ | 40 mM |
| [OsCl(Him)(dmbpy)₂]Cl₂ | 0.3 mM |
| PIPES (pH 7.0) | 50 mM |
| CHAPS | 0.1 wt% |

### Example 10

A copper complex was prepared by using various ligands. Copper (II) chloride and each of the following ligands were mixed at a molar ratio of 1 : 2, dissolved in purified water, and incubated for 10 minutes in a water bath at about 80°C so that the ligands were coordinated to the metal. Thus, complex solutions were obtained.

| Ligand | Manufacturer | Complex |
|---|---|---|
| 1, 10-phenanthroline | Wako Pure Chemical Industries, Ltd. | [Cu(1,10-phenanthroline)₂] |
| bathophenanthroline | Wako Pure Chemical Industries, Ltd. | [Cu(bathophenanthroline)₂] |
| bathophenanthroline sulfonic acid disodium salt | Nacalai Tesque, Inc. | [Cu(bathophenanthroline sulfonic acid)₂] |
| 2,2'-bipyridyl | Wako Pure Chemical Industries, Ltd. | [Cu(2,2'-bipyridyl)₂] |
| TPTZ | DOJINDO LABORATORIES | [Cu(TPTZ)₂] |
| cuproin | Wako Pure Chemical Industries, Ltd. | [Cu(cuproin)₂] |

### Example 11

A copper mixed ligand complex was prepared by using each of the following ligands and the bipyridyl compounds. Copper, each of the following ligands, and the bipyridyl compounds were mixed at a molar ratio of 1 : 2 : : 1, dissolved in purified water, and incubated for 10 minutes in a water bath at about 80°C so that the ligands and the bipyridyl compounds were coordinated to the metal. Thus, complex solutions were obtained.

| Ligand | Manufacturer | Complex |
|---|---|---|
| L-arginine | Nacalai Tesque, Inc. | [Cu(L-Arg)(bpy)] |
| ethylenediamine | Nacalai Tesque, Inc. | [Cu(en)(bpy)] |
| imidazole | Wako Pure Chemical Industries, Ltd. | [Cu(Him)(bpy)] |

### Example 12

An iron complex was prepared by using various ligands. Iron (III) chloride and each of the following ligands were mixed at a molar ratio of 1 : 3, dissolved in purified water, and incubated for 10 minutes in a water bath at about 80°C so that the ligands were coordinated to the metal. Thus, complex solutions were obtained.

| Ligand | Manufacturer | Complex |
|---|---|---|
| 1,10-phenanthroline | Wako Pure Chemical Industries, Ltd. | [Fe(1,10-phenanthroline)₃] |
| bathophenanthroline | Wako Pure Chemical Industries, Ltd. | [Fe(bathophenanthroline)₃] |
| bathophenanthroline sulfonic acid disodium salt | Nacalai Tesque, Inc. | [Fe(bathophenanthroline sulfonic acid)₃] |
| 2,2'-bipyridyl | Wako Pure Chemical Industries, Ltd. | [Fe(2,2'-bipyridyl)₃] |
| 4,4'-diamino-2,2'-bipyridyl (DA-bpy) | Arkray, Inc. | [Fe(4,4'-diamino-2,2'-bipyridyl)₃] |
| TPTZ | DOJINDO LABORATORIES | [Fe(TPTZ)₃] |
| PDTS | DOJINDO LABORATORIES | [Fe(PDTS)₃] |
| nitroso-PSAP | DOJINDO LABORATORIES | [Fe(nitroso-PSAP)₃] |
| nitroso-ESAP | DOJINDO LABORATORIES | [Fe(nitroso-ESAP)₃] |
| 1-nitroso-2-naphthol | KANTO KAGAKU | [Fe(1-nitroso-2-naphthol)₃] |
| 2-amino-4-thiazole acetic acid | Lancaster | [Fe(2-amino-4-thiazole acetic acid)₃] |
| 1,2-naphthoquinone-4-sulfonic acid | Nacalai Tesque, Inc. | [Fe(1,2-naphthoquinone-4-sulfonic acid)₃] |
| nitro-PAPS | DOJINDO LABORATORIES | [Fe(nitro-PAPS)₃] |

### Example 13

Two types of ruthenium complexes were prepared in the following manner. [Ru(NH₃)₆]

A commercially available ruthenium complex (manufactured by Aldrich, Hexaammineruthenium (III) chloride) was dissolved in water to obtain a complex solution of [Ru(NH₃)₆].
[Ru(4,4'-diamino-2,2'-bipyridyl)₃]

### Ligand

First, 11.8 g (63.0 mmol) of 2,2'-bipyridyl-N,N'-dioxide (manufactured by Aldrich) was dissolved slowly in 120 ml of concentrated sulfuric acid cooled in an ice bath, and the solution was heated to 100°C. Then, a concentrated sulfuric acid solution (100 ml) containing 64.0 g (630 mmol) of potassium nitrate was slowly added dropwise and stirred for 1 hour while heating. After reaction, the solution was cooled to room temperature, poured into crushed ice, and filtered. Thus, a solid of 4,4'-dinitro-2,2'-bipyridyl-N,N'-oxide was obtained. Next, 7.0 g (25 mM) of 4,4'-dinitro-2,2'-bipyridyl-N,N'-oxide and 6.0 g of 10% palladium carbon were suspended in ethanol (23 ml) under Ar. To this solution was added dropwise an ethanol solution (47 ml) containing 6.3 g (126 mmol) of hydrazine monohydrate, followed by refluxing for 8 hours. The solution was cooled and filtered. The filtrate was concentrated and purified by silica gel column chromatography. Thus, 4,4'-diamino-2,2'-bipyridyl was obtained.

### Synthesis

Ethylene glycol (10 mL) was placed in a 50 mL two-neck flask, in which DA-bpy (0.2 g) and RuCl₃ (0.1 g) were dissolved and stirred successively. The solution was heated by a mantle heater while vigorously stirring under N₂, followed by refluxing for about 4 hours. Purification

After stirring and cooling under N₂, the solution was moved to a 100 mL round bottom flask and washed with acetone (5 mL) + diethyl ether (20 mL). This washing of the solution with acetone (5 mL) + diethyl ether (20 mL) was repeated until the solvent (ethylene glycol) was removed sufficiently. The target substance thus washed was dissolved in ethanol and precipitated by the addition of diethyl ether. The target substance was filtered while washing with diethyl ether and dried under reduced pressure. Thus, a solid of [Ru(4,4'-diamino-2,2'-bipyridyl)₃] was obtained. This solid was dissolved in water to obtain a complex solution.

### Example 14

Two types of osmium complexes were prepared in the following manner.
[OsCl(Him)(dmbpy)₂]

### Synthesis

First, 2.00 g (4.56 mmol) of (NH₄)₂[OsCl₆] (manufactured by Aldrich) and 1.68 g (9.11 mmol) of 4,4'-dimethyl-2,2'-bipyridyl (dmbpy, manufactured by Wako Pure Chemical Industries, Ltd.) were refluxed in ethylene glycol (60 ml) for 1 hour under N₂. After cooling to room temperature, 1M sodium dithionite solution (120 ml) was added for 30 minutes, followed by cooling in an ice bath for 30 minutes. The precipitates were filtered under reduced pressure and sufficiently washed with water (500 to 1000 ml). Further, the precipitates were washed with diethyl ether two times, and then dried under reduced pressure. Thus, 1.5 to 1.7 g of [OsCl₂(dmbpy)₂] was obtained. Next, 1.56 g (2.60 mmol) of [OsCl₂(dmbpy)₂] and 0.36 g (5.2 mmol) of imidazole (Him) were refluxed in a water/methanol mixed solvent (50 ml) under N₂ for 2 hours. After cooling to room temperature, a saturated NaCl solution (300 ml) was added. The precipitates were filtered under reduced pressure, washed with a saturated NaCl solution, and dried under reduced pressure. Thus, [OsCl(Him)(dmbpy)₂]Cl₂ was obtained.

### Purification

The [OsCl(Him)(dmbpy)₂]Cl₂ was dissolved in the smallest possible amount of acetonitrile/methanol (1 : 1 v/v) and purified by column chromatography (absorbent: activated alumina, developing solvent: acetonitrile/methanol). The solvent was evaporated, and the residue was dissolved in a small amount of acetone and reprecipitated with diethyl ether. The precipitates were filtered and dried under reduced pressure, and then dissolved in water. Thus, a complex solution was obtained.
(Os(Him)₂(dmbpy)₂]

### Synthesis

First, 2.00 g (4.56 mmol) of (NH₄)₂[OsCl₆] and 1.68 g (9.11 mmol) of dmbpy were refluxed in ethylene glycol (60 ml) under N₂ for 1 hour. After cooling to room temperature, 1M sodium dithionite solution (120 ml) was added for 30 minutes, followed by cooling in an ice bath for 30 minutes. The precipitates were filtered under reduced pressure and sufficiently washed with water (500 to 1000 ml). Further, the precipitates were washed with diethyl ether two times, and then dried under reduced pressure. Thus, 1.5 to 1.7 g of [OsCl₂(dmbpy)₂] was obtained. Next, 1.56 g (2.60 mmol) of (OsCl₂(dmbpy)₂] and 0.36 g (5.2 mmol) of imidazole (Him) were refluxed in a 1,2-ethanedithiol solvent (50 ml) under N₂ for 2 hours. After cooling to room temperature, a saturated NaCl solution (300 ml) was added. The precipitates were filtered under reduced pressure, washed with a saturated NaCl solution, and dried under reduced pressure. Thus, [Os(Him)₂(dmbpy)₂]Cl₂ was obtained.

### Purification

The [Os(Him)₂(dmbpy)₂]Cl₂ was dissolved in the smallest possible amount of acetonitrile/methanol (1 : 1 v/v) and purified by column chromatography (absorbent: activated alumina, developing solvent: acetonitrile/methanol). The solvent was evaporated, and the residue was dissolved in a small amount of acetone and reprecipitated with diethyl ether. The precipitates were filtered and dried under reduced pressure, and then dissolved in water. Thus, a complex solution was obtained.

### Example 15

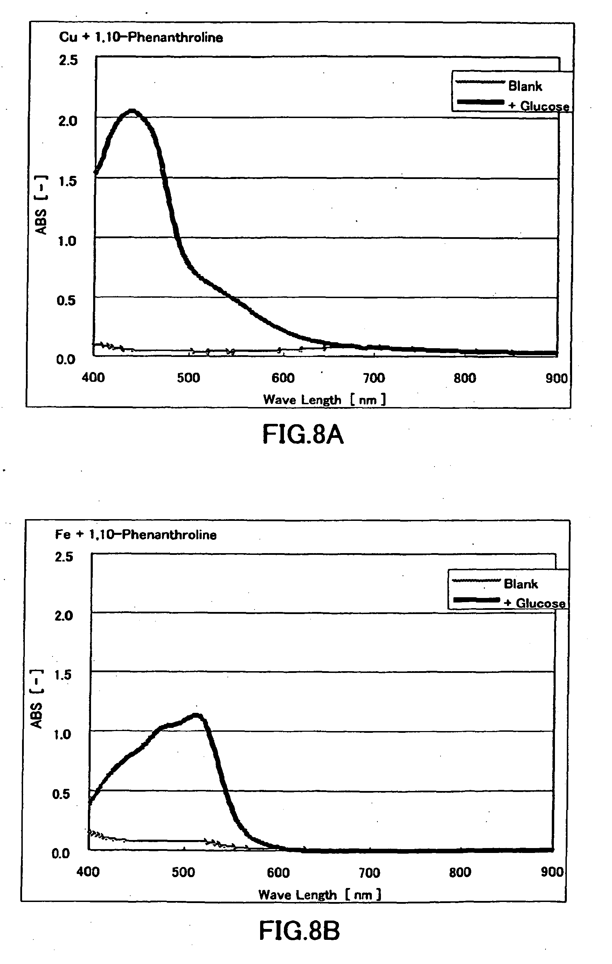

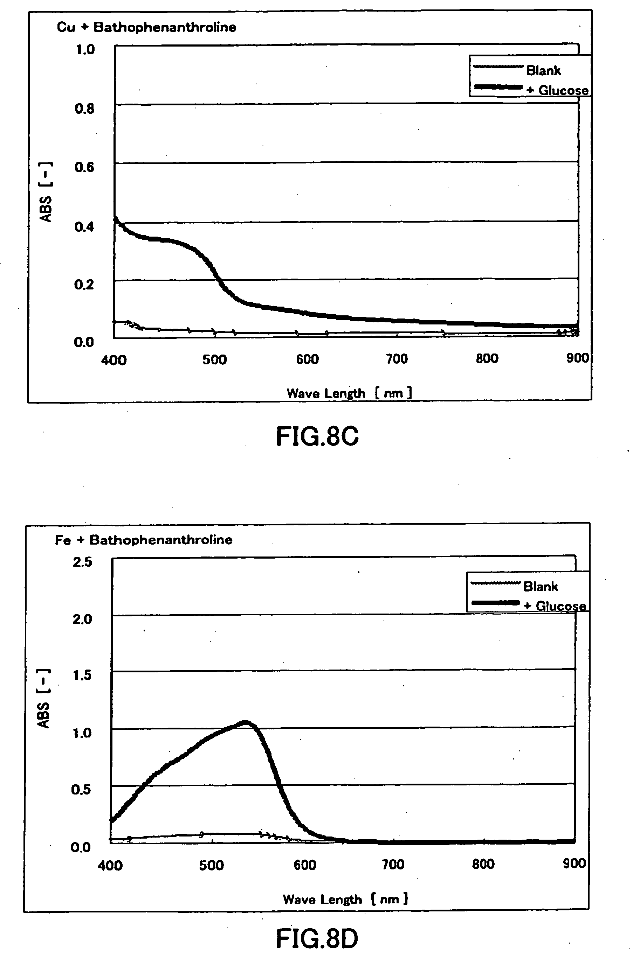

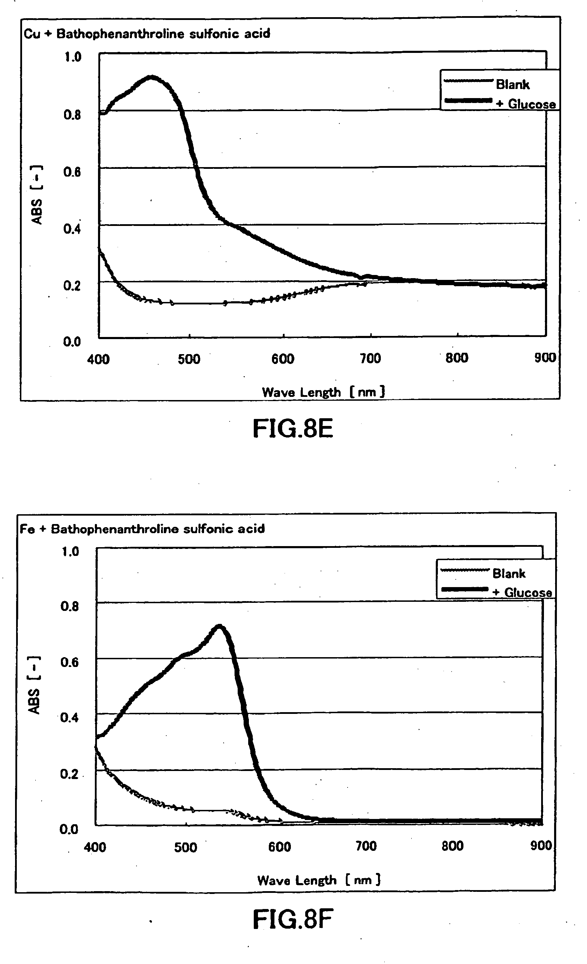

Reagent solutions were prepared by mixing a complex including phenanthroline ligands in NN coordination form, an enzyme, and a buffer solution with the following compositions 1 to 6. The spectrum of each of the reagent solutions was measured and identified as a blank. Further, glucose equivalent in amount to the complex was added to each of the reagent solutions, and the spectrum was measured after the color change. FIGS. 8A to 8F show the results. The complexes prepared in the above examples were used.

| Reagent solution composition 1 (FIG. 8A) | |
|---|---|
| PQQ-GDH | 50 U/mL |
| [Cu(1,10-phenanthroline)₂] | 1 mM |
| PIPES pH 7 | 50 mM |
| Triton X-100 | 0.5% |

| Reagent solution composition 2 (FIG. 8B) | |
|---|---|
| PQQ-GDH | 50 U/mL |
| [Fe(1,10-phenanthroline)₃] | 0.1mM |
| PIPES pH 7 | 50 mM |
| Triton X-100 | 0.5% |

| Reagent solution composition 3 (FIG. 8C) | |
|---|---|
| PQQ-GDH | 50 U/mL |
| [Cu(bathophenanthroline)₂] | 1mM |
| PIPES pH 7 | 50 mM |
| Triton X-100 | 0.5% |
| (bathophenanthroline = 4,7-diphenyl phenanthroline) | |

| Reagent solution composition 4 (FIG. 8D) | |
|---|---|
| PQQ-GDH | 50 U/mL |
| [Fe(bathophenanthroline)₃] | 1 mM |
| PIPES pH 7 | 50 mM |
| Triton X-100 | 0.5% |
| (bathophenanthroline = 4,7-diphenyl phenanthroline) | |

| Reagent solution composition 5 (FIG. 8E) | |
|---|---|
| PQQ-GDH | 50 U/mL |
| (Cu(bathophenanthroline sulfonic acid)₂] | 1 mM |
| PIPES pH 7 | 50 mM |
| Triton X-100 | 0.5% |

| Reagent solution composition (FIG. 8F) | |
|---|---|
| PQQ-GDH | 50 U/mL |
| [Fe(bathophenanthroline sulfonic acid)₃] | 0.1 mM |
| PIPES pH 7 | 50 mM |
| Triton X-100 | 0.5% |

### Example 16

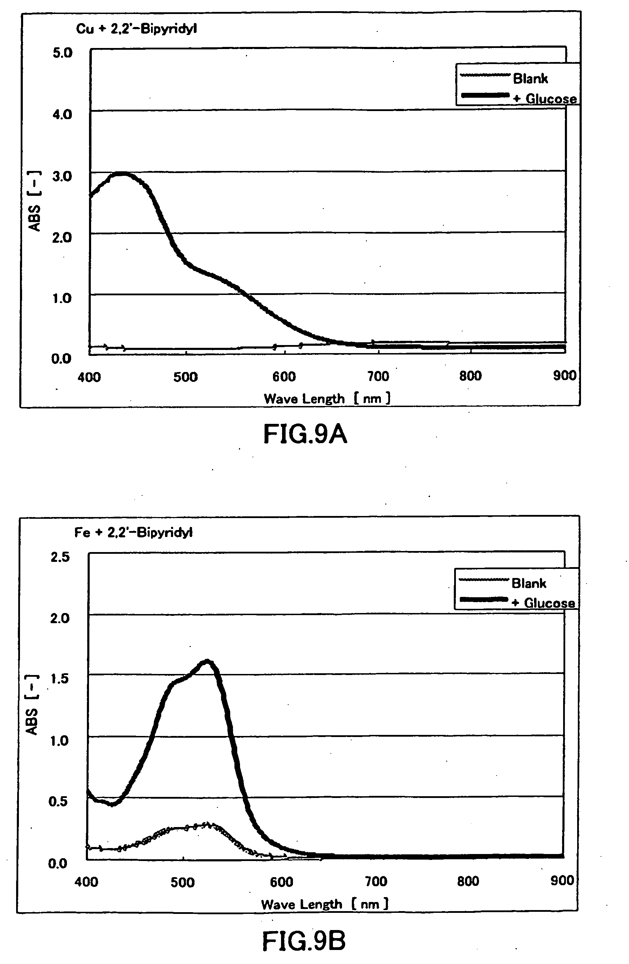

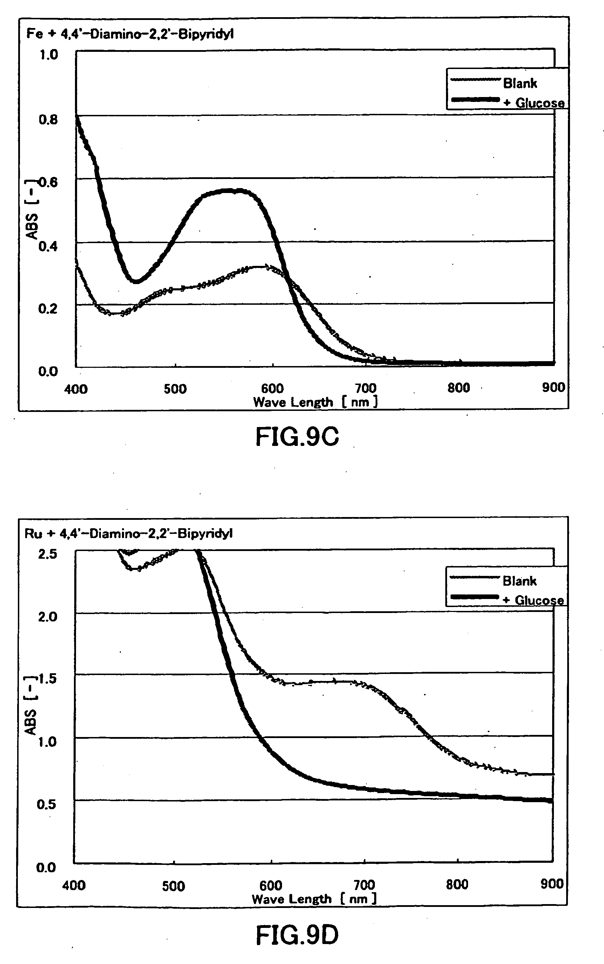

Reagent solutions were prepared by mixing a complex including bipyridyl ligands in NN coordination form, an enzyme, and a buffer solution with the following compositions 1 to 4. The spectrum of each of the reagent solutions was measured and identified as a blank. Further, glucose equivalent in amount to the complex was added to each of the reagent solutions, and the spectrum was measured after the color change. FIGS. 9A to 9D show the results. The complexes prepared in the above examples were used.

| Reagent solution composition 1 (FIG. 9A) | |
|---|---|
| PQQ-GDH | 50 U/mL |
| [Cu(2,2'-bipyridyl)₂] | 1 mM |
| PIPES pH 7 | 50 mM |
| Triton X-100 | 0.5% |

| Reagent solution composition 2 (FIG. 9B) | |
|---|---|
| PQQ-GDH | 50 U/mL |
| [Fe(2,2'-bipyridyl)₃] | 1 mM |
| PIPES pH 7 | 50 mM |
| Triton X-100 | 0.5% |

| Reagent solution composition 3 (FIG. 9C) | |
|---|---|
| PQQ-GDH | 50 U/mL |
| [Fe(4,4'-diamino-2,2'-bipyridyl)₃] | 0.1 mM |
| PIPES pH 7 | 50 mM |
| Triton X-100 | 0.5% |

| Reagent solution composition 4 (FIG. 9D) | |
|---|---|
| PQQ-GDH | 50 U/mL |
| [Ru(4,4'-diamino-2,2'-bipyridyl)₃] | 10 mM |
| PIPES pH 7 | 50 mM |
| Triton X-100 | 0.5% |

### Example 17

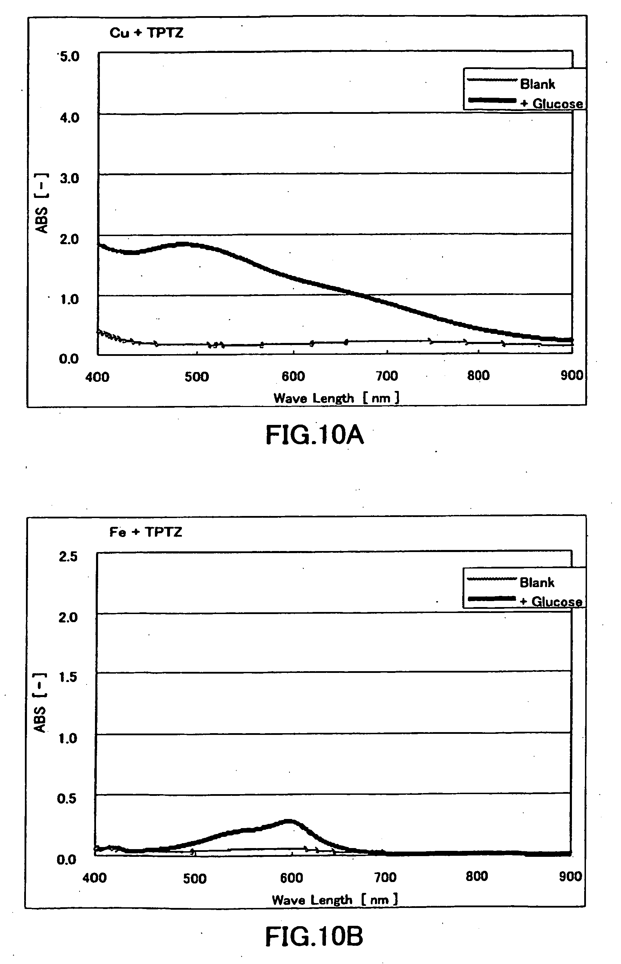

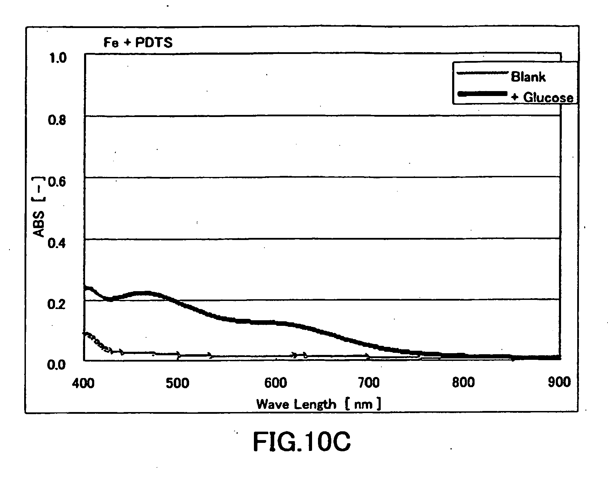

Reagent solutions were prepared by mixing a complex including triazine ligands in NN coordination form, an enzyme, and a buffer solution with the following compositions 1 to 3. The spectrum of each of the reagent solutions was measured and identified as a blank. Further, glucose equivalent in amount to the complex was added to each of the reagent solutions, and the spectrum was measured after the color change. FIGS. 10A to 10C show the results. The complexes prepared in the above examples were used.

| Reagent solution composition 1 (FIG. 10A) | |
|---|---|
| PQQ-GDH | 50 U/mL |
| [Cu(TPTZ)₂] | 1 mM |
| PIPES pH 7 | 50 mM |
| Triton X-100 | 0.5% |
| (TPTZ = 2,4,6-tripyridyl-s-triazine) | |

| Reagent solution composition 2 (FIG. 10B) | |
|---|---|
| PQQ-GDH | 50 U/mL |
| [Fe(TPTZ)₃] | 0.1 mM |
| PIPES pH 7 | 50 mM |
| Triton X-100 | 0.5% |
| (TPTZ = 2,4,6-tripyridyl-s-triazine) | |

| Reagent solution composition 3 (FIG. 10C) | |
|---|---|
| PQQ-GDH | 50 U/mL |
| [Fe(PDTS)₃] | 1 mM |
| PIPES pH 7 | 50 mM |
| Triton X-100 | 0.5% |
| (PDTS = 3-(2-pyridyl)-5,6-bis(4 sulphophenyl-1,2,4-triazine) | |

### Example 18

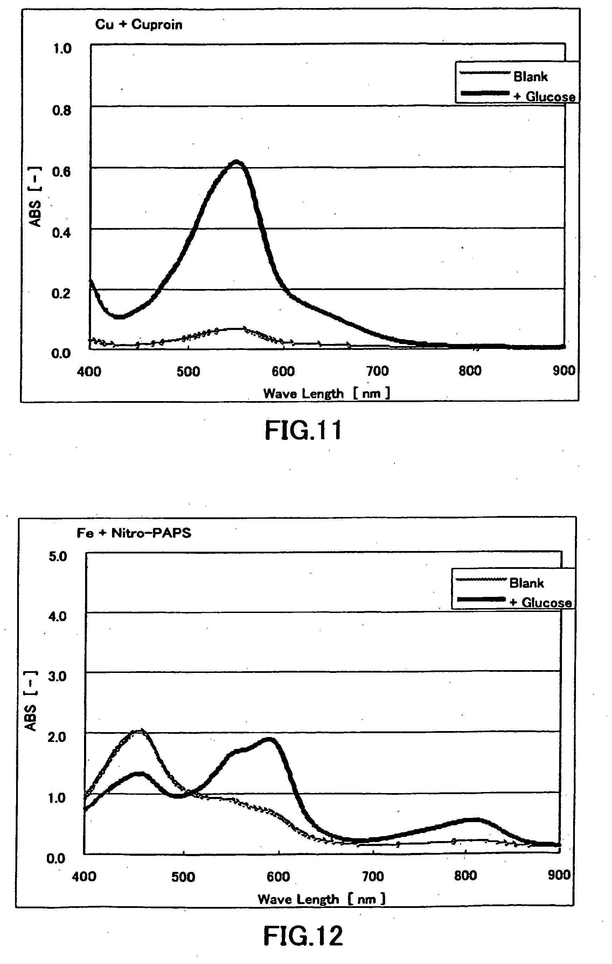

A reagent solution was prepared by mixing a complex including biquinoline ligands in NN coordination form, an enzyme, and a buffer solution with the following composition. The spectrum of the reagent solution was measured and identified as a blank. Further, glucose equivalent in amount to the complex was added to the reagent solution, and the spectrum was measured after the color change. FIG. 11 shows the results. The complex prepared in the above examples was used.

| Reagent solution composition | |
|---|---|
| PQQ-GDH | 50 U/mL |
| [Cu(cuproin)₂] | 1 mM |
| PIPES pH 7 | 50 mM |
| Triton X-100 | 0.5% |

| | |
|---|---|
| (cuproin = 2,2'-biquinoline) | |

### Example 19

A reagent solution was prepared by mixing a complex including pyridylazo ligands in NN coordination form, an enzyme, and a buffer solution with the following composition. The spectrum of the reagent solution was measured and identified as a blank. Further, glucose equivalent in amount to the complex was added to the reagent solution, and the spectrum was measured after the color change. FIG. 12 shows the results. The complex prepared in the above examples was used.

| Reagent solution composition | |
|---|---|
| PQQ-GDH | 50 U/mL |
| [Fe(nitro-PAPS)₃] | 0.02 mM |
| PIPES pH 7 | 50 mM |
| Triton X-100 | 0.5% |

| | |
|---|---|
| (nitro-PAPS = 2-(5-nitro-2-pyridylazo)-5-[N-n-propyl-N-(3-sulfopropyl)aminophenol]) | |

### Example 20

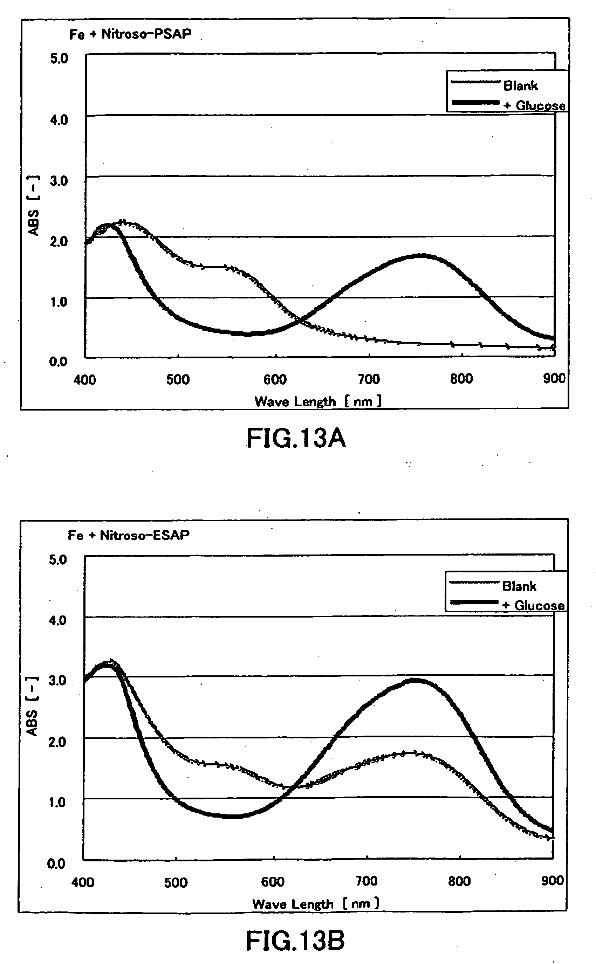

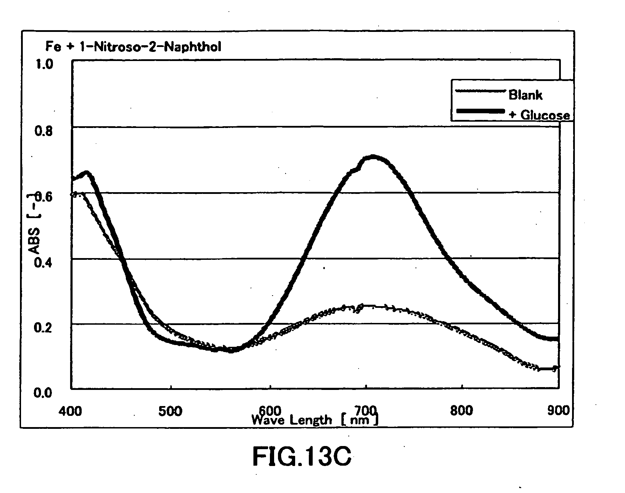

Reagent solutions were prepared by mixing a complex including ligands in NO coordination form, an enzyme, and a buffer solution with the following compositions 1 to 3. The spectrum of each of the reagent solutions was measured and identified as a blank. Further, glucose equivalent in amount to the complex was added to each of the reagent solutions, and the spectrum was measured after the color change. FIGS. 13A to 13C show the results. The complexes prepared in the above examples were used.

| Reagent solution composition 1 (FIG. 13A) | |
|---|---|
| PQQ-GDH | 50 U/mL |
| [Fe(nitroso-PSAP)₃] | 0.05 mM |
| PIPES pH 7 | 50 mM |
| Triton X-100 | 0.5% |
| (nitroso-PSAP = | |
| 2-nitroso-5-[N-n-propyl-N-(3-sulfopropyl)aminophenol]) | |

| Reagent solution composition 2 (FIG. 13B) | |
|---|---|
| PQQ-GDH | 50 U/mL |
| [Fe(nitroso-ESAP)₃] | 0.1 mM |
| PIPES pH 7 | 50 mM |
| Triton X-100 | 0.5% |
| (nitroso-ESAP = 2-nitroso-5-[N-ethyl-N-(3-sulfopropyl)aminophenol]) | |

| Reagent solution composition 3 (FIG. 13C) | |
|---|---|
| PQQ-GDH | 50 U/mL |
| [Fe(1-nitroso-2-naphthol)₃] | 0.1 mM |
| PIPES pH 7 | 50 mM |
| Triton X-100 | 0.5% |

### Example 21

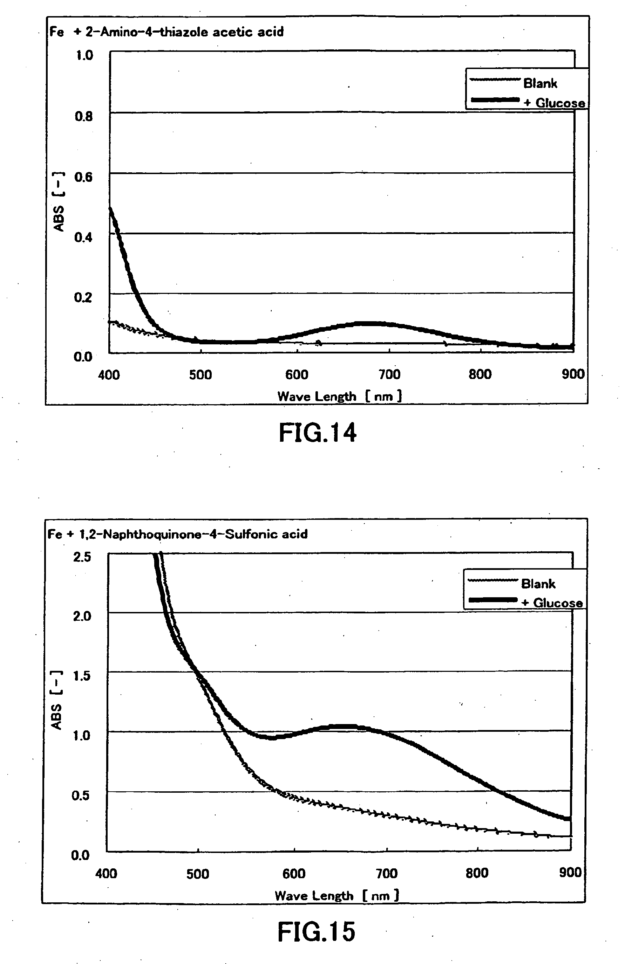

A reagent solution was prepared by mixing a complex including ligands in NS coordination form, an enzyme, and a buffer solution with the following composition. The spectrum of the reagent solution was measured and identified as a blank. Further, glucose equivalent in amount to the complex was added to the reagent solution, and the spectrum was measured after the color change. FIG. 14 shows the results. The complex prepared in the above examples was used.

| Reagent solution composition | |
|---|---|
| PQQ-GDH | 50 U/mL |
| [Fe(2-amino-4-thiazoleacetic acid)₃] | 1 mM |
| PIPES pH 7 | 50 mM |
| Triton X-100 | 0.5% |

### Example 22

A reagent solution was prepared by mixing a complex including ligands in OO coordination form, an enzyme, and a buffer solution with the following composition. The spectrum of the reagent solution was measured and identified as a blank. Further, glucose equivalent in amount to the complex was added to the reagent solution, and the spectrum was measured after the color change. FIG. 15 shows the results. The complex prepared in the above examples was used.

| Reagent solution composition | |
|---|---|
| PQQ-GDH | 50 U/mL |
| [Fe(1,2-naphthoquinone-4-sulfonic acid)₃] | 1 mM |
| PIPES pH 7 | 50 mM |
| Triton X-100 | 0.5% |

### Example 23

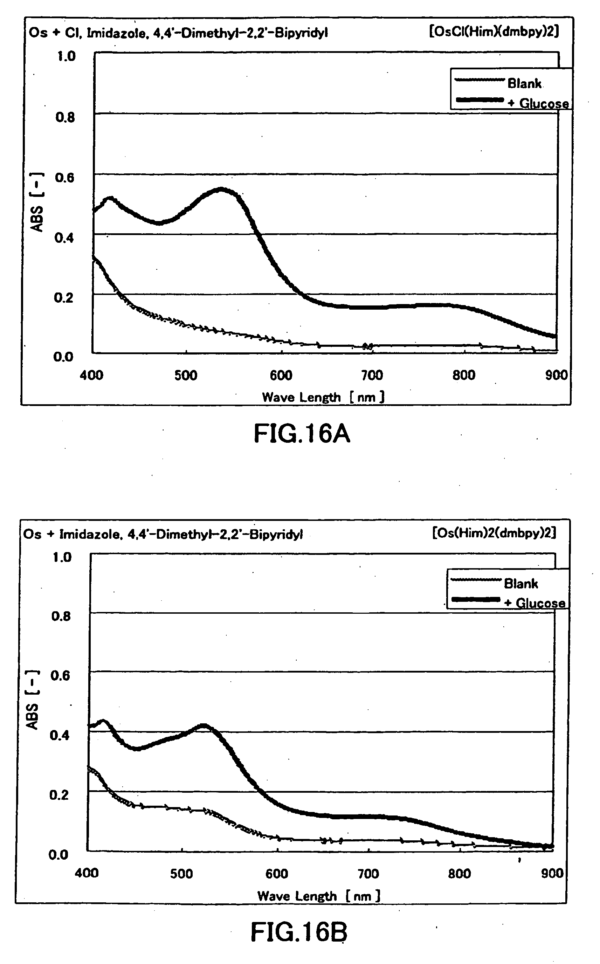

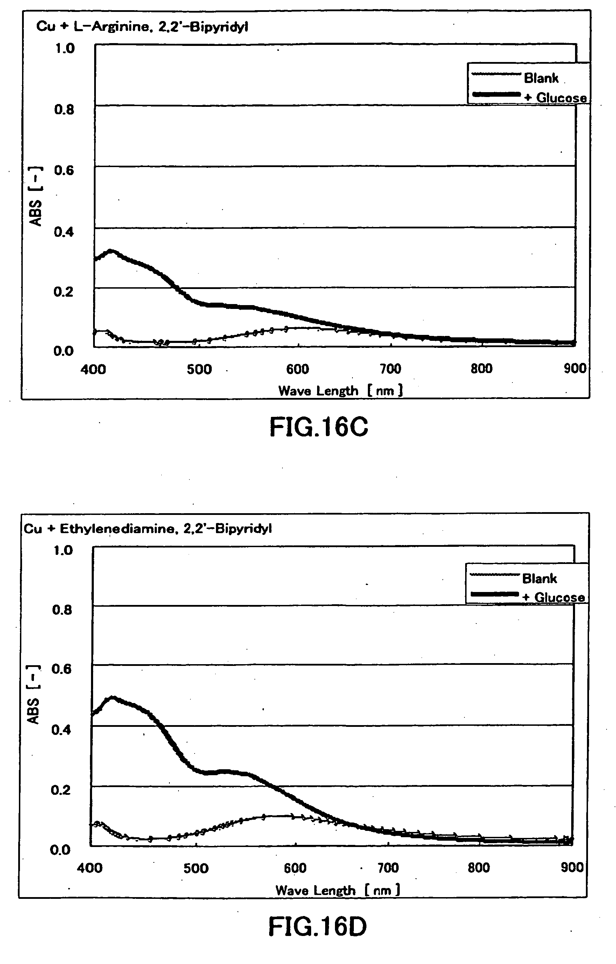

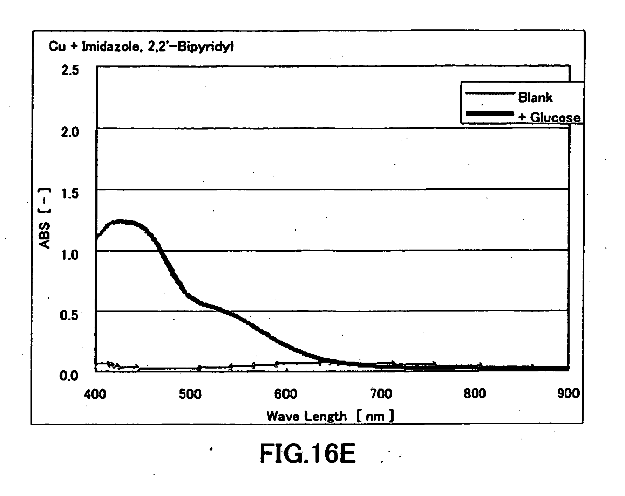

Reagent solutions were prepared by mixing a mixed ligand complex, an enzyme, and a buffer solution with the following compositions 1 to 5. The spectrum of each of the reagent solutions was measured and identified as a blank. Further, glucose equivalent in amount to the complex was added to each of the reagent solutions, and the spectrum was measured after the color change. FIGS. 16A to 16E show the results. The complexes prepared in the above examples were used.

| Reagent solution composition 1 (FIG. 16A) | |
|---|---|
| PQQ-GDH | 50 U/mL |
| [OsCl(Him)(dmbpy)₂] | 0.1 mM |
| PIPES pH 7 | 50 mM |
| Triton X-100 | 0.5% |
| (Him = imidazole) | |
| (dmbpy = 4,4'-dimethyl-2,2'-bipyridyl) | |

| Reagent solution composition 2 (FIG. 16B) | |
|---|---|
| PQQ-GDH | 50 U/mL |
| [Os(Him)₂(dmbpy)₂] | 0.1 mM |
| PIPES pH 7 | 50 mM |
| Triton X-100 | 0.5% |

| Reagent solution composition 3 (FIG. 16C) | |
|---|---|
| PQQ-GDH | 50 U/mL |
| [Cu(L-Arg)₂(bpy)] | 1 mM |
| PIPES pH 7 | 50 mM |
| Triton X-100 | 0.5% |
| (L-Arg = L-arginine) | |
| (bpy = 2,2'-bipyridyl) | |

| Reagent solution composition 4 (FIG. 16D) | |
|---|---|
| PQQ-GDH | 50 U/mL |
| [Cu(en)₂(bpy)] | 1 mM |
| PIPES pH 7 | 50 mM |
| Triton X-100 | 0.5% |
| (en = ethylenediamine) | |
| (bpy = 2,2'-bipyridyl) | |

| Reagent solution composition 5 (FIG. 16E) | |
|---|---|
| PQQ-GDH | 50 U/mL |
| [Cu(Him)₂(bpy)] | 1 mM |
| PIPES pH 7 | 50 mM |
| Triton X-100 | 0.5% |

### Example 24

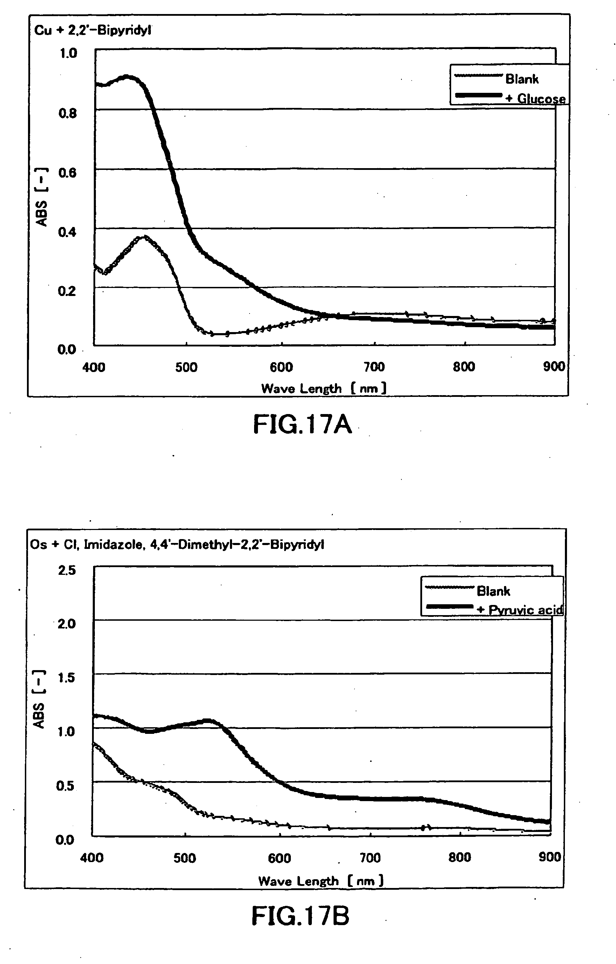

Reagent solutions were prepared by mixing a complex, an enzyme (glucose oxidase (GOD) or pyruvate oxidase), and a buffer solution with the following compositions 1 and 2. The spectrum of each of the reagent solutions was measured and identified as a blank. Further, glucose or pyruvic acid equivalent in amount to the complex was added to each of the reagent solutions, and the spectrum was measured after the color change. FIGS. 17A and 17B show the results. The complexes prepared in the above examples were used.

| Reagent solution composition 1 (FIG. 17A) | |
|---|---|
| GOD | 100 U/mL |
| [Cu(2,2'-bipyridyl)₂] | 1 mM |
| PIPES pH 7 | 50 mM |
| Triton X-100 | 0.5% |

| Reagent solution composition 2 (FIG. 17B) | |
|---|---|
| pyruvate oxidase | 100 U/mL |
| [OsCl(Him)(dmbpy)₂] | 0.1 mM |
| PIPES pH 7 | 50 mM |
| Triton X-100 | 0.5% |
| (Him = imidazole) | |
| (dmbpy = 4,4'-dimethyl-2,2'-bipyridyl) | |

### Example 25

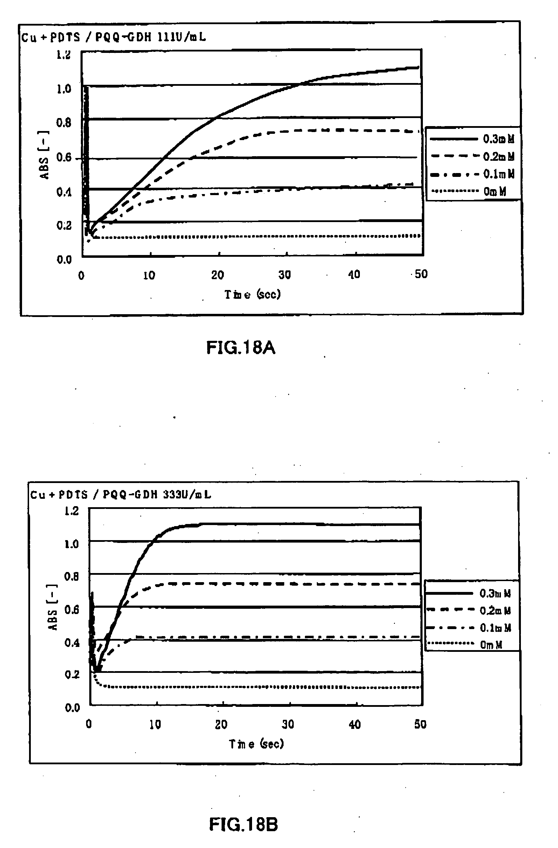

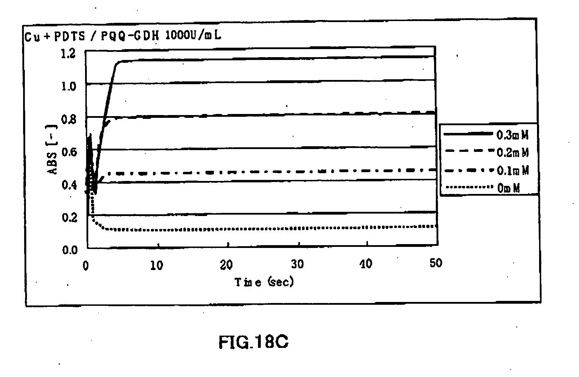

A reagent solution was prepared with the following composition. Moreover, the following enzyme solutions 1, 2, and 3 were prepared. Then, 10 µL of glucose aqueous solution (with concentrations of 0, 10, 20, and 30 mM and final concentrations of 0, 0.1, 0.2, and 0.3 mM, respectively) and 500 µL of the reagent solution were mixed in a dispocell having an optical path length of 10 mm, to which 500 µL of each of the enzyme solutions was added to cause a reaction between the solutions. The absorbance change was measured for 50 seconds with a spectrophotometer (wavelength: 600 nm). FIGS. 18A, 18B, and 18C show the results. As shown in FIGS. 18A, 18B, and 18C, the reaction rate increased with the amount of enzyme. When the enzyme activity was 1000 U/mL, the reaction came to an end in about 5 seconds. It is possible to quantify the glucose concentration by sampling signals near 5 seconds, at which the reaction reaches the end. The slope of the graphs from the beginning to the end of the reaction also can be used to quantify the glucose concentration.

| Reagent solution composition | |
|---|---|
| Cu(PDTS)₂ | 1 mM |
| PIPES pH 7 | 50 mM |
| Triton X-100 | 0.5% |
| Enzyme solution 1 (FIG. 18A) | |
| PQQ-GDH | 111 U/mL |
| Enzyme solution 2 (FIG. 18B) | |
| PQQ-GDH | 333 U/mL |
| Enzyme solution 3 (FIG. 18C) | |
| PQQ-GDH | 1000 U/mL |

### Industrial Applicability

As described above, a colorimetric method of the present invention can perform simple and reliable analysis in a short time.

## Claims

1. A colorimetric method comprising:
allowing an oxidoreductase to act on an analyte and a transition metal complex that changes color by transferring an electron; and
performing a qualitative or quantitative analysis of the analyte by measuring a color change in the transition metal complex due to electron transfer from the analyte to the transition metal complex,
wherein
at least one ligand in the transition metal complex is selected from ammonia, a bipyridyl compound, an imidazole compound, a phenanthroline compound, an ethylenediamine compound, amino acids, a triazine compound, a biquinoline compound, a pyridylazo compound, a nitroso compound and an oxine compound.

2. The method according to claim 1, wherein the transition metal complex comprises at least one complex selected from a copper complex, an iron complex, a ruthenium complex, and an osmium complex.

3. The method according to claim 1 or claim 2, wherein at least one hydrogen atom that occupies a position other than a coordination position of the ligand is replaced by a substituent.

4. The method according to claim 3, wherein the substituent is selected from an alkyl group, an aryl group, an allyl group, a phenyl group, a hydroxyl group, an alkoxy group, a carboxyl group, a carbonyl group, a sulfone group, a sulfonyl group, a nitro group, a nitroso group, a primary amine, a secondary amine, a tertiary amine, an amino group, an acyl group, an amido group, and a halogen group.

5. The method according to any one of claims 1 to 4, wherein the transition metal complex includes two or more types of ligands.

6. The method according to any one of claims 1 to 5, wherein the oxidoreductase is a dehydrogenase or an oxidase.

7. The method according to any one of claims 1 to 6, wherein the analyte is glucose, cholesterol, uric acid, pyruvic acid, lactic acid, creatine or creatinine, and the oxidoreductase is a dehydrogenase or an oxidase that corresponds to the respective analyte.

8. The method according to any one of claims 1 to 7 wherein a mediator is used in addition to the transition metal complex.

## Patentansprüche

1. Kolorimetrisches Verfahren, das umfasst:
Einwirkenlassen einer Oxidoreduktase auf einen Analyten und einen Übergangsmetallkomplex, der seine Farbe durch Elektronentransfer bzw. Transfer eines Elektrons ändert; und
Durchführen einer qualitativen oder quantitativen Analyse des Analyten durch Messen einer Farbveränderung in dem Übergangsmetallkomplex aufgrund von Elektronentransfer von dem Analyten auf den Übergangsmetallkomplex,
wobei
wenigstens ein Ligand in dem Übergangsmetallkomplex aus Ammoniak, einer Bipyridylverbindung, einer Imidazolverbindung, einer Phenanthrolinverbindung, einer Ethylendiaminverbindung, Aminosäuren, einer Triazinverbindung, einer Bichinolinverbindung, einer Pyridylazoverbindung, einer Nitrosoverbindung und einer Oxinverbindung ausgewählt ist.

2. Verfahren nach Anspruch 1, bei dem der Übergangsmetallkomplex wenigstens einen Komplex umfasst, der aus einem Kupferkomplex, einem Eisenkomplex, einem Rutheniumkomplex und einem Osmiumkomplex ausgewählt ist.

3. Verfahren nach Anspruch 1 oder 2, bei dem wenigstens ein Wasserstoffatom, das eine Stelle bzw. Position besetzt, die keine Koordinationsstelle des Liganden ist, durch einen Substituenten ersetzt ist.

4. Verfahren nach Anspruch 3, bei dem der Substituent ausgewählt ist aus einer Alkylgruppe, einer Arylgruppe, einer Allylgruppe, einer Phenylgruppe, einer Hydroxylgruppe, einer Alkoxygruppe, einer Carboxylgruppe, einer Carbonylgruppe, einer Sulfongruppe, einer Sulfonylgruppe, einer Nitrogruppe, einer Nitrosogruppe, einem primären Amin, einem sekundären Amin, einem tertiären Amin, einer Aminogruppe, einer Acylgruppe, einer Amidogruppe und einer Halogengruppe.

5. Verfahren nach einem der Ansprüche 1 bis 4, bei dem der Übergangsmetallkomplex zwei oder mehrere Arten von Liganden enthält.

6. Verfahren nach einem der Ansprüche 1 bis 5, bei dem die Oxidoreduktase eine Dehydrogenase oder eine Oxidase ist.

7. Verfahren nach einem der Ansprüche 1 bis 6, bei dem der Analyt Glukose, Cholesterin, Harnsäure, Brenztraubensäure, Milchsäure, Kreatin oder Kreatinin ist und die Oxidoreduktase eine Dehydrogenase oder eine Oxidase ist, die dem jeweiligen Analyten entspricht.

8. Verfahren nach einem der Ansprüche 1 bis 7, bei dem ein Mediator zusätzlich zu dem Übergangsmetallkomplex verwendet wird.

## Revendications

1. Méthode colorimétrique comprenant les étapes consistant à :
➢ laisser une oxydoréductase réagir sur un analyte et un complexe de métal de transition qui change de couleur en transférant un électron ; et
➢ effectuer une analyse quantitative ou qualitative de l'analyte en mesurant un changement de couleur dans le complexe de métal de transition, dû au transfert d'électron de l'analyte vers le complexe de métal de transition,
dans laquelle
au moins un ligand dans le complexe de métal de transition est choisi parmi l'ammoniac, un composé de bipyridyle, un composé d'imidazole, un composé de phénanthroline, un composé d'éthylènediamine, des acides aminés, un composé de triazine, un composé de biquinoline, un composé pyridylazo, un composé nitroso et un composé d'oxine.

2. Méthode selon la revendication 1, dans laquelle le complexe de métal de transition comprend au moins un complexe choisi parmi un complexe de cuivre, un complexe de fer, un complexe de ruthénium, et un complexe d'osmium.

3. Méthode selon la revendication 1 ou 2, dans laquelle au moins un atome d'hydrogène qui occupe une position différente d'une position de coordination du ligand est remplacé par un substituant.

4. Méthode selon la revendication 3, dans laquelle le substituant est choisi parmi un groupe alkyle, un groupe aryle, un groupe allyle, un groupe phényle, un groupe hydroxyle, un groupe alcoxy, un groupe carboxyle, un groupe carbonyle, un groupe sulfone, un groupe sulfonyle, un groupe nitro, un groupe nitroso, une amine primaire, une amine secondaire, une amine tertiaire, un groupe amino, un groupe acyle, un groupe amido, et un groupe halogène.

5. Méthode selon l'une quelconque des revendications 1 à 4, dans laquelle le complexe de métal de transition comprend deux types de ligands ou plus.

6. Méthode selon l'une quelconque des revendications 1 à 5, dans laquelle l'oxydoréductase est une déshydrogénase ou une oxydase.

7. Méthode selon l'une quelconque des revendications 1 à 6, dans laquelle l'analyte est le glucose, le cholestérol, l'acide urique, l'acide pyruvique, l'acide lactique, la créatine ou créatinine, et l'oxydoréductase est une déshydrogénase ou une oxydase qui correspond à l'analyte respectif.

8. Méthode selon l'une quelconque des revendications 1 à 7, dans laquelle un médiateur est utilisé en plus du complexe de métal de transition.
